(19) Europäisches Patentamt
European Patent Office
Office européen des brevets

(11) EP 2 237 665 B1

(12) # FASCICULE DE BREVET EUROPEEN

(45) Date de publication et mention de la délivrance du brevet:
**30.10.2013 Bulletin 2013/44**

(21) Numéro de dépôt: **09707004.9**

(22) Date de dépôt: **30.01.2009**

(51) Int Cl.:
***A01N 37/38*** *(2006.01)* ***C07C 51/42*** *(2006.01)*
***A01N 37/36*** *(2006.01)* ***A01P 7/04*** *(2006.01)*

(86) Numéro de dépôt international:
**PCT/FR2009/050137**

(87) Numéro de publication internationale:
**WO 2009/095624 (06.08.2009 Gazette 2009/32)**

(54) **PROCEDE DE PREPARATION D'ACIDES DICAFEOYLQUINIQUES ET LEUR UTILISATION DANS LA LUTTE CONTRE LES PUCERONS**

VERFAHREN ZUR HERSTELLUNG VON DICAFFEOYLCHINASÄUREN UND IHRE VERWENDUNG ZUR BEKÄMPFUNG VON BLATTLÄUSEN

METHOD FOR PREPARING DICAFFEOYLQUINIC ACIDS AND USE THEREOF IN COMBATING APHIDS

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **01.02.2008 FR 0800561**

(43) Date de publication de la demande:
**13.10.2010 Bulletin 2010/41**

(73) Titulaire: **Institut National de la Recherche Agronomique**
**75338 Paris Cedex 07 (FR)**

(72) Inventeurs:
- **POËSSEL, Jean-Luc**
**F-84250 Le Thor (FR)**
- **COLLET, Marie-Hélène**
**F-84450 Saint Saturnin Les Avignon (FR)**
- **RAHBE, Yves, Noël, Elie**
**F-69100 Villeurbanne (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
- **CLIFFORD W. BENINGER ET AL.: "A flavanone and two phenolic acids from Chrysamthemum morifolium with phytotoxic and insect growth regulating activity" JOURNAL OF CHEMICAL ECOLOGY, vol. 30, no. 3, 2004, pages 589-606, XP007905930 cité dans la demande**
- **DATABASE CA [Online] CHEMICAL ABSTRACTS SERVICE, COLUMBUS, OHIO, US; 2006, JIN, HAILI ET AL: "Tissue cultures and polyphenol production in Solidago altissima L" XP002580153 extrait de STN Database accession no. 2007: 252417 & NIPPON SHOKUHIN KAGAKU GAKKAISHI , 13(3), 136-140 CODEN: NSKGF4; ISSN: 1341-2094, 2006,**
- **HOOK I L I: "Leontopodium alpinum Cass. (Edelweiss): In vitro culture, micropropagation, and the production of secondary metabolites" BIOTECHNOLOGY IN AGRICULTURE AND FORESTRY, SPRINGER VERLAG, BERLIN, DE, 1 janvier 1993 (1993-01-01), pages 217-232, XP009130585 ISSN: 0934-943X**
- **DATABASE CA [Online] CHEMICAL ABSTRACTS SERVICE, COLUMBUS, OHIO, US; 2002, KABGANIAN, R. ET AL: "Localization of alkamides, echinacoside and cynarin in Echinacea angustifolia" XP002580154 extrait de STN Database accession no. 2003:664580 & JOURNAL OF HERBS, SPICES & MEDICINAL PLANTS , 10(2), 73-81 CODEN: JHEPEF; ISSN: 1049-6475, 2002,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **YOSHIHIKO MARUTA ET AL: "ANTIOXIDTIVE CAFFEOYLQUINIC ACID DERIVATIVES IN THE ROOTS OF BURDOCK (ARCTIUM LAPPA L.)" JOURNAL OF AGRICULTURAL AND FOOD CHEMISTRY, AMERICAN CHEMICAL SOCIETY, SCIENTIFIC PUBLISHERS, NEW DELHI - INDIA LNKD- DOI:10.1021/JF00058A007, vol. 43, no. 10, 1 octobre 1995 (1995-10-01), pages 2592-2595, XP000535986 ISSN: 0021-8561**
- **ALKHATIB R ET AL: "Activity of elaeochytrin A from Ferula elaeochytris on leukemia cell lines" PHYTOCHEMISTRY, PERGAMON PRESS, GB LNKD- DOI:10.1016/J.PHYTOCHEM.2008.09.019, vol. 69, no. 17, 1 décembre 2008 (2008-12-01), pages 2979-2983, XP025681590 ISSN: 0031-9422 [extrait le 2008-11-05]**
- **DOS SANTOS M D ET AL: "Analgesic activity of di-caffeoylquinic acids from roots of Lychnophora ericoides (Arnica da serra)" JOURNAL OF ETHNOPHARMACOLOGY, ELSEVIER SCIENTIFIC PUBLISHERS LTD, IE LNKD- DOI:10.1016/J.JEP.2004.09.043, vol. 96, no. 3, 15 janvier 2005 (2005-01-15), pages 545-549, XP004689956 ISSN: 0378-8741**
- **A.O.TAYLOR AND MILTON ZUCKER: "Turnover and metabolism of chlorogenic acid in Xanthium leaves and potato tubers" PLANT PHYSIOLOGY, vol. 41, 1966, pages 1350-1359, XP002580155**
- **MAKIKO TAKENAKA ET AL.: "Caffeic acid derivatives in the roots of yacon (Smallanthus sonchifolius)" JOURNAL OF AGRICULTURAL AND FOOD CHEMISTRY, vol. 51, 2003, pages 793-796, XP002580156**


Remarques:

Le dossier contient des informations techniques présentées postérieurement au dépôt de la demande et ne figurant pas dans le présent fascicule.

## Description

La présente invention a pour objet la préparation de l'acide 3, 5-dicaféoylquinique, et de certains de ses dérivés, et leur utilisation dans la préparation de produits phytosanitaires.

L'acide 3, 5-dicaféoylquinique (3, 5-diCQ) et ses différents dérivés ont été identifiés chez de nombreuses espèces d'intérêt agronomique appartenant à différentes familles botaniques (rosacées dont les arbres fruitiers à pépins et à noyau ; solanacées dont la tomate et la pomme de terre ; astéracées dont le tournesol, l'artichaut et la laitue ; rubiacées dont le café ; convolvulacées dont la patate douce....).

Plusieurs centaines de publications décrivent la présence de ces substances chez ces différentes espèces et mettent en évidence leurs propriétés chimiques et biologiques.

Ces composés présentent une forte activité antioxydante, qui leur confère un grand intérêt d'un point de vue nutritionnel (Ohnishi et al, (1994) Phytochemistry, 36: 579-583 ; Iwai et al, (2004) Journal of Agricultural and Food Chemistry, 52: 4893-4898 ; Kim & Lee, (2005) Planta Medica, 71: 871-876; Saito et al ; (2005) Bioorganic & Medicinal Chemistry, 13: 4191-4199).

Ils montrent également de multiples propriétés médicinales, entre autres en tant qu'analgésiques (dos Santos et al, (2005) Journal of Ethnopharmacology 96: 545-549), antihypertenseurs (Mishima et al, (2005a) Biological & Pharmaceutical Bulletin 28: 1909-1914), hypouricémiants (Nguyen et al, (2005) Biological & Pharmaceutical Bulletin 28: 2231-2234), hépatoprotecteurs (Basnet et al, (1996) Biol Pharm Bull 19: 1479-1484), anti-inflammatoires (Peluso et al, (1995) Journal of Natural Products 58: 639-646) et anticancéreux (Mishima et al, (2005b) Bioorganic & Medicinal Chemistry 13: 5814-5818).

Ces composés sont présents dans des préparations utilisées en médecine traditionnelle, dans des extraits végétaux actuellement commercialisés (extraits à base d'artichaut ou d'échinacée) et dans la propolis (Mishima et al, (2005a) Biological & Pharmaceutical Bulletin 28: 1909-1914 ; Mishima et al, (2005b) Bioorganic & Medicinal Chemistry 13: 5814-5818 ; Basnet et al, (1996) Biol Pharm Bull 19: 1479-1484). Ils possèdent également d'importantes propriétés anti-infectieuses, notamment antivirales. Ils constituent une nouvelle classe d'antirétrovirus, en particulier pour le traitement du SIDA, par leur action inhibitrice sur l'intégrase du HIV (Mahmood et al, (1993) Antiviral Chem. Chemother. 4: 235-240 ; Mc Dougall et al, (1998) Antimicrobial Agents & Chemotherapy 42: 140-146 ; Zhu et al, (1999) J Virol 73: 3309-3316).

Le brevet EP1008344 décrit l'utilisation de ce groupe de molécules dans le traitement de l'hépatite B, la demande internationale WO2006127525 leur utilisation dans le traitement du sida, la demande japonaise JP2006213636 préconise l'utilisation de ce groupe de molécules dans le traitement de cancers, la demande EP0577516 décrit des compositions dermatologiques dépigmentantes qui les contiennent et la demande EP 1312373 évoque leurs effets anti-allergiques.

Les propriétés fongicides de ces molécules ont également été fréquemment montrées (Bazzalo et al, (1985) Phytopathologische Zeitschrift 112: 322-332 ; Kodoma et al, (1998) Phytochemistry 47: 371-373 ; Stange et al, (2001) Postharvest Biology & Technology 23: 85-92).

En revanche, les propriétés insecticides ont été beaucoup plus rarement étudiées et les résultats sont très discordants. Bien que certains auteurs aient établi une corrélation entre la teneur élevée en diCQ des racines de cultivars de laitue et leur résistance à un aphidé (*Pemphigus bursarius*), ils n'ont jamais montré l'activité biologique directe de ces molécules vis à vis de l'insecte (Cole et al. (1984) Annals of Applied Biology 105: 129-145). D'autres auteurs ont montré que certains dérivés présentaient peu ou pas d'activité insecticide chez des insectes broyeurs (Beninger et al, (2004) J Chem Ecol 30: 589-605 ; Schwarz et al 1996). Certains auteurs ont même montré un effet phagostimulant de 3, 5-diCQ sur des insectes broyeurs (Tamura et al. (2004) ; Mullin et al (1991) ;

Le pêcher *Prunus persica* (L.) Batsch (Rosacées), arbre pafficulièrement bien adapté au climat méditerranéen, est principalement cultivé en Chine, en Amérique du nord (Californie), en Amérique du sud (Chili et Argentine) et en Europe dans les pays du pourtour du Bassin Méditerranéen où les principaux centres de culture sont l'Espagne, l'Italie, la Grèce et la France. En 2004, la production mondiale de pêches était de 15, 6 millions de tonnes, répartie sur une surface de 1, 4 millions d'hectares. La production française cette même année était de 413 000 tonnes produites principalement le long de la Vallée du Rhône, en Provence, dans le Roussillon et le long de la Vallée de la Garonne. Cette culture représente pour ces régions méridionales un enjeu économique incontestable.

Cependant, le pêcher peut être la cible de nombreuses attaques parasitaires d'origine animale ou microbienne. Les principaux pathogènes et ravageurs dont l'hôte est le pêcher sont des champignons: l'oïdium (*Sphaerotheca pannosa*), la cloque (*Tophrina deformans*) et la moniliose (*Monilinia laxa, M fructigena et M fructicola*); des bactéries pouvant provoquer un dépérissement (*Xanthomonas arboricola* pathovar *pruni*) et de nombreux insectes et virus.

Une attaque particulièrement préoccupante est celle du puceron vert du pêcher, *Myzus persicae* (Sulzer). Cet insecte piqueur suceur est particulièrement nuisible, non seulement par les dégâts directs qu'il occasionne mais aussi parce qu'il est un vecteur potentiel du Plum Pox Virus, agent causal de la maladie de la Sharka, qui provoque des déformations ainsi que des décolorations des fruits, les rendant alors non commercialisables. Aucun moyen de lutte

curative n'étant disponible, les arbres infectés doivent être arrachés.

Pour lutter contre ce parasite, l'agriculteur se trouve alors confronté à deux problématiques :

- la lutte chimique contre le puceron vert qui exige des insecticides puissants (souvent nocifs et toxiques) et dont l'efficacité est par ailleurs aléatoire, entraînant un risque d'apparition de résistance chez *Myzus persicae* (Guillemaud et al, (2003) Bulletin of Entomological Research **93**, 289-297) et
- la prise en compte du souci de l'environnement, qui vise par ailleurs à réduire les intrants phytosanitaires.

Cette problématique a amené généticiens et entomologistes à chercher à améliorer la résistance du pêcher vis-à-vis de *Myzus persicae.*

Le puceron vert du pêcher appartient à l'ordre des Hémiptères et à la famille des Aphididae. Il mesure de 1, 2 à 2, 5 mm de long. C'est un puceron phytophage qui se nourrit de sève élaborée, prélevée au niveau du phloème par des pièces buccales de type piqueur-suceur (Hullé et al, (1998) dans ACTA et INRA éditions, 77 pages).

Le cycle annuel de *Myzus persicae,* sous climat tempéré, se compose d'une phase de reproduction sexuée, conduisant à la ponte d'un oeuf d'hiver diapausant, suivie d'une phase de reproduction parthénogénétique (cycle complet ou holocyclie). Ces deux phases ont lieu sur des plantes hôtes différentes (cycle dioécique), appelées respectivement hôte primaire (le pêcher) et hôte secondaire (plantes herbacées dont certaines ont une importance économique majeure : pomme de terre, choux, aubergine, betterave...).

Les oeufs d'hiver pondus à la base des bourgeons de pêcher éclosent de fin janvier à fin avril, lors du débourrement et donnent des femelles parthénogenétiques aptères, les fondatrices. Ces fondatrices sont à l'origine de plusieurs générations de femelles parthénogenétiques, les *fondatrigènes* sur l'hôte primaire. Dès la fin du printemps et en été, lorsqu'il y a surpopulation sur le pêcher, les *fondatrigènes* donnent naissance à des individus ailés qui colonisent leur hôte secondaire. Ces individus ailés engendreront par reproduction asexuée plusieurs générations d'aptères ou d'ailés, les *virginogènes,* qui à l'automne donneront des individus mâles et femelles sexués et ailés, les *sexupares.* Ces individus retourneront sur le pêcher pour pondre les oeufs d'hiver (Hullé et al, (1998) déjà cité ; Sauge, (1999) Analyse des mécanismes de résistance du pêcher *Prunus persica* (L.) Batsch au puceron vert Thèse Université Pierre et Marie Curie (Paris 6) 188 pages).

Les dégâts directs causés par ce puceron sont dus aux piqûres alimentaires de celui-ci qui provoquent le dessèchement des bourgeons, la chute des fleurs, des déformations du feuillage et des jeunes pousses, perturbent la croissance et peuvent induire des réactions nécrotiques (Massonié et al, (1979) Revue de zoologie agricole et pathologie végétale 78, 1-5 ; Monet et Massonié, (1994) Agronomie 2 177-182 ; Monet et Guye, (1998) in Monet R. (Ed) Proc. Fourth Intern. Peach Symposium Acta Hort 171-175). De plus, *Myzus persicae,* comme mentionné précédemment, est un vecteur du plum pox virus (provoquant la maladie de la sharka), appartenant au groupe des potyvirus qui provoquent des dégâts indirects, décolorations des feuilles et des déformations des fruits.

Compte tenu de ce qui précède il est nécessaire de trouver de nouveaux traitements pour lutter de manière efficace mais respectueuse de l'environnement contre les pucerons, notamment le puceron vert du pêcher (*Myzus persicae*).

Or, les inventeurs ont mis en évidence l'effet répulsif et toxique du 3, 5-diCQ et de certains de ses dérivés sur différentes espèces de pucerons.

Cependant, le 3, 5-diCQ et ses isomères, à part le 1, 3-diCQ (ou cynarine), isolée de l'artichaut, ne sont pas aisément disponibles commercialement. Aussi ces composés doivent-ils être extraits de différents végétaux. Outre l'artichaut, on peut citer le café (EP0299107) et le tournesol chez qui on a décrit la surproduction de composés phénoliques et plus particulièrement d'acides dicaféoylquiniques en utilisant un éliciteur biotique, le pathogène *Sclerotinia* (EP1671535).

D'autres sources de 3, 5-diCQ et de ses dérivés sont les racines de *Solidago altissima* (Nippon Shokuhin Kagaku Gakkaishi, 2006, 13(3), 136-140), *Leontopodium alpinum* (Biotechnology in Agriculture and Forestry, 1993, vol. 21, pages 217-232), *Echinacea angustifolia* (Journal of Herbs, Spices & Medicinal Plants , 2002, 10(2), 73-81), *Arcticum lappa* (Journal of Agricultural and Food Chemistry, 1995, vol. 43(10), pages 2592-2595), *Ferula elaeochytris* (Phytochemistry, 2008, vol. 69(17), pages 2979-2983) et *Lychnophora ericoides* (Journal of Ethnopharmacology, 2005, vol. 96(3), pages 545-549).

Il existe donc un besoin important de développer des procédés d'obtention de 3, 5-diCQ et de ses dérivés faciles à mettre en oeuvre et présentant un fort rendement.

Les inventeurs ont donc cherché à purifier cette molécule en quantité suffisante. L'isolement du 3, 5-diCQ et ses dérivés à partir du pêcher n'apparaissait pas satisfaisant en raison des teneurs relativement faibles de la substance et de la présence de nombreux composés phénoliques pouvant entraver la purification (figure 1). En revanche *Ipomoea batatas* (convolvulacées), dont la composition phénolique a été étudiée en particulier dans le tubercule comestible (patate douce) et les feuilles par des équipes japonaises (Islam et al, (2002) Journal of Agricultural and Food Chemistry 50: 371-8-3722 ; Islam et al (2003) Journal of Food Science 68: 111-116) s'est révélée être une source prometteuse.

Les racines non tubérisées d'*Ipomoea batatas* ont révélé une teneur importante en 3, 5-diCQ (environ 15 mg par g de matière sèche) et une faible teneur en formes isomères contaminantes (figure 2). Ces teneurs sont particulièrement importantes dans des conditions de carence minérale, en particulier en azote, et de culture des racines à la lumière. Les teneurs peuvent atteindre 90 mg par g de matière sèche en culture sur eau distillée à la lumière.

Aussi la présente invention a pour objet un procédé de préparation de composés de formule (I)

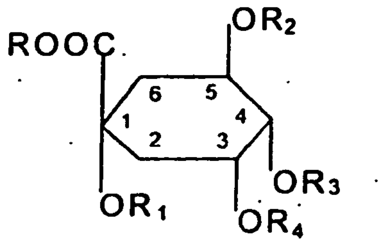

dans laquelle

- R représente un atome d'hydrogène ou un groupement méthyle,
- $R_1$, $R_2$ et $R_4$ représentent chacun indépendamment l'un de l'autre un atome d'hydrogène ou un groupe caféoyle et
- $R_3$ représente un hydrogène, un groupe caféoyle ou un groupe succinyle, à condition que deux au moins parmi $R_1$ à $R_4$ représentent un groupe caféoyle, et que $R_3$ représente un groupement succinyle seulement si $R_2$ et $R_4$ représentent un groupe caféoyle,

à partir de racines non tubérisées comprenant les étapes suivantes :

a) prélèvement des racines non tubérisées ou récupération de l'exsudat racinaire,
b) extraction par un ou plusieurs solvants organiques des composés phénoliques,
c) récupération de l'extrait brut,
d) purification éventuelle dudit extrait obtenu à l'étape c),
e) optionnellement, isomérisation spontanée dudit extraits en condition de pH alcalin, lesdits composés de formule (I) pouvant être sous la forme de régio ou stéréoisomères, ou de leurs mélanges, les racines non tubérisées étant issues de plantes du genre *Ipomoea* (convolvulacées) ou des genres de la même famille *Argyreia, Calycobolus, Calystegia, Convolvulus, Dichondra, Erycibe, Evolvulus, Iseia*, *Jacquemontia, Maripa, Merremia, Mina, Operculina, Porana, Stictocardia,* ou *Turbina.*

On entend par racine non tubérisée une racine de structure normale, excluant les parties présentant le phénomène de tubérisation qui se caractérisé par l'accumulation de réserves et l'épaississement de la racine et conduit à la formation d'un tubercule consommable. Dans le cas d'*Ipomoea batalas,* le tubercule consommable est couramment dénommé patate douce.

On entend par groupement caféoyle, le groupement représente par la formule suivante

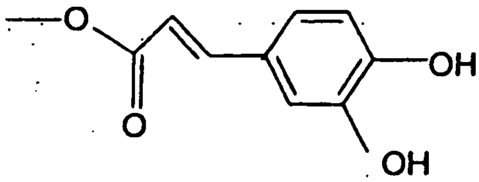

Ces composés sont extraits de la racine non tubérisée d'une plante du genre *Ipomoea* (convolvulacées) ou des genres de la même famille *Argyreia, Calycobolus, Calystegia, Convolvulus, Dichondra, Erycibe, Evolvulus, Iseia, Jacquemontia, Maripa, Merremia, Mina, Operculina, Porana, Stictocardia,* ou *Turbina.* Outre *Ipomoea batatas,* le genre *Ipomoea* comprend environ 500 espèces dont des plantes ornementales comme le volubilis (*Ipomoea purpurea*) ou d'usage alimentaire comme le KangKong ou liseron d'eau (*Ipomoea aquatica*) consommé en Asie. Le genre *Convolvulus* comprend environ 250 espèces dont le liseron des champs (*Convolvulus arvensis*) et la belle de jour (*Convolvulus tricolor*). Le genre *Calystegia* comprend environ 25 espèces dont le liseron des haies (*Calystegia sepia*) et le liseron des bois (*Calystegia silvatica*).

Particulièrement, l'acide 3, 5-dicaféoylquinique et ses dérivés sont extraits des racines non tubérisées d'une plante choisie dans le groupe comprenant la patate douce (*Ipomoea batatas*), le volubilis (*Ipomoea purpurea*), le liseron

d'eau (*Ipomoea aquatica*), le volubilis des jardins (*Ipomoea indica*), l'Ipomée Scarlett O'Hara (*Ipomoca nil*) et le liseron des haies (*Calystegi sepium).*

Dans un mode avantageux de réalisation du procédé de l'invention, l'acide 3, 5-dicaféoylquinique et ses dérivés sont extraits des racines non tubérisées d'*Ipomoea batatas.*

Les racines non tubérisées d'*Ipomoea batatas* peuvent être produites à partir de semis, de boutures, de marcottes ou de tubercules, cultivés en culture hydroponique selon la technique décrite dans la demande internationale WO 01/33942, sur substrat horticole ou en pleine terre, avantageusement en condition de carence minérale. Elles peuvent être récolées dès que leur quantité est suffisante pour procéder à une extraction du 3, 5-diCQ.

Avantageusement, les racines non tubérisées sont produites par culture en milieu liquide, à la lumière et en situation de carence minérale.

Dans un mode avantageux de l'invention, les composés de formule (I),

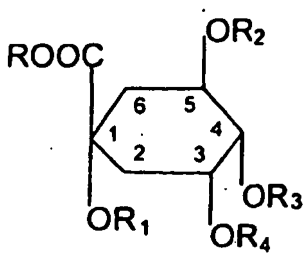

qui sont préparés, sont choisis parmi l'acide 3, 5-dicaféoylquinique (3, 5-diCQ), les différentes formes isomères de 3, 5-diCQ, notamment la cynarine (1, 3-diCQ), l'acide 1, 5-dicaféoylquinique (1, 5-diCQ), l'acide 3, 4-dicaféoylquinique (3, 4-diCQ) et l'acide 4, 5-dicaféoylquinique (4, 5-diCQ), ses analogues triacylés comme l'acide 3, 4, 5-tricaféoylquinique (3, 4, 5-triCQ), ses analogues méthylés comme le méthyle 3, 5-dicaféoylquinate, le méthyle 3, 4-dicaféoylquinate, le méthyle 4, 5-dicaféoylquinate, l'acide 4-succinyl-3, 5-dicaféoylquinique.

Dans un mode de réalisation particulièrement avantageux de l'invention, le procédé de préparation des composés de formule (I), notamment de l'acide 3, 5-dicaféoylquinique comprend les étapes suivantes :

a) prélèvement des racines d'*Ipomoea batatas,* issues de tubercules, boutures, semis ou marcottes,
b) congélation dans l'azote liquide des racines prélevées à l'étape a),
c) lyophilisation des racines congelées à l'étape b),
d) broyage dans l'azote liquide des racines lyophilisées puis lyophilisation pour obtenir une poudre sèche,
e) extraction des composés phénoliques par un solvant organique en 1 à 4 fois, par agitation à froid,
f) rinçage du dernier résidu de l'étape e) par le même solvant organique que celui utilisé à l'étape e) puis évaporation du solvant présent dans l'extrait jusqu'à obtention d'une phase aqueuse,
g) optionnellement, extraction liquide/liquide volume à volume par un solvant apolaire, par plusieurs extractions successives après addition d'un sel et d'un acide dans la phase aqueuse pour faciliter l'extraction des diCQ dans le solvant apolaire,
h) concentration à sec de la phase aqueuse obtenue à l'étape f) ou de la phase organique obtenue à l'étape optionnelle g) après adjonction d'un agent de séchage pour éliminer l'eau résiduelle et filtration, puis reprise du résidu sec par un solvant organique,
i) séparation par CLHP semi-préparative en phase inverse et collecte de la fraction contenant le 3,5-diCQ,
j) concentration de la fraction obtenue à l'étape i) jusqu'à l'obtention d'une phase aqueuse,
k) extraction liquide/liquide par un solvant organique (volume à volume) par plusieurs extractions successives après addition d'un sel, dans la phase aqueuse pour faciliter l'extraction dans le solvant apolaire de 3,5 diCQ,
l) concentration à sec de la phase organique, après adjonction d'un agent de séchage, et filtration pour éliminer l'eau résiduelle, et reprise par un solvant organique,
m) précipitation au froid de la molécule par ajout d'eau (au moins 3 volumes pour 1 volume de solvant organique utilisé à l'étape précédente), congélation de l'extrait dans l'azote liquide et lyophilisation pour obtenir la molécule sous forme de poudre sèche.

Dans un mode de réalisation avantageux de l'invention, les solvants organiques utilisés à l'étape e) sont ceux classiquement utilisés dans l'extraction des plantes ; ils sont notamment choisis dans le groupe comprenant l'éthanol, le méthanol, et l'acétone, ces solvants pouvant être mélangés avec de l'eau, comme par exemple l'éthanol à 70% utilisé dans l'exemple 1.

Dans un mode de réalisation particulièrement avantageux de l'invention, l'étape g) est réalisée par extraction

par l'acétate d'éthyle ou le diéthyléther (volume à volume) après addition de NaCl ou de sulfate d'ammonium (de 8 % à saturation) et d'acide métaphosphorique 1-2% poids/volume final), selon des protocoles adaptés de Macheix (1974) Thèse Doctorat Sciences Naturelles, Université Paris, VI, 168 p..

Les étapes d'évaporation des solvants sont réalisées par toute technique classiquement utilisées, notamment à l'aide d'un évaporateur rotatif et l'agent de séchage utilisé aux étapes h) et l) est avantageusement du sulfate de sodium anhydre.

Le solvant organique utilisé à l'étape h) est en général celui utilisé pour la chromatographie semi-préparative de l'étape i) et est avantageusement du méthanol de pureté CLHP.

La chromatographie semi-préparative de l'étape i) est réalisée avantageusement sur colonne C18 par un gradient eau acidifiée à pH 2, 6/méthanol.

Le procédé selon l'invention permet d'obtenir l'acide 3, 5-dicaféoylquinique avec une pureté supérieure à 90 % avantageusement comprise entre 93 et 95 %. Les substances contaminantes sont essentiellement des isomères du 3, 5-diCQ.

Dans un mode de réalisation avantageux de l'invention, pour mieux conserver lors de l'extraction, les formes isomères natives présentes initialement dans les tissus végétaux, l'ensemble des étapes a) à m) est réalisé à froid, de préférence à une température comprise entre 3 et 5 °C, à l'abri d'une exposition prolongée à la lumière et le pH acide des solvants aqueux est de préférence compris entre 5, 0 et 6, 0.

Les acides 3, 4-dicaféoylquinique (3, 4-diCQ) et 4, 5-dicaféoylquinique (4, 5-diCQ) peuvent être facilement produits à partir de l'acide 3, 5-dicaféoylquinique par isomérisation spontanée en condition de pH alcalin. Ainsi l'isomérisation du 3, 5-diCQ peut être réalisée dans un tampon Tris-HCl (50 mM) pH 8, 5 pendant 2 heures à 40°C selon une méthode adaptée de Hanson (1965) Biochemistry 4: 2719-2731, et Moller et Herrmann (1982) Journal of Chromatography 241 : 371-379. Dans ces conditions les trois formes isomères sont présentes en teneurs comparables et peuvent être séparées par CLHP semi-préparative selon un protocole identique à celui décrit précédemment pour la purification du 3, 5-diCQ.

La présente invention a également pour objet l'utilisation d'au moins un composé de formule (I) ou de leur mélange pour lutter contre les pucerons.

Selon l'invention, les composés de formule (I) sont utilisés sous forme de composition comprenant une quantité efficace desdits composés.

Au sens de composition comprenant une quantité efficace d'au moins un composé choisi parmi l'acide 3, 5-dicaféoylquinique (3, 5-diCQ), les différentes formes isomères de 3, 5-diCQ, notamment la cynarine (1, 3-diCQ), l'acide 1, 5-dicaféoylquinique (1, 5-diCQ), l'acide 3, 4-dicaféoylquinique (3, 4-diCQ) et l'acide 4, 5-dicaféoylquinique (4, 5-diCQ), ses analogues triacylés comme l'acide 3, 4, 5-tricaféoylquinique (3, 4, 5-triCQ), ses analogues méthylés comme le méthyl 3, 5-dicaféoylquinate, l'acide 4-succinyl-3, 5-dicaféoylquinique, on entend soit des extraits bruts, soit des extraits purifiés, lesdits extraits pouvant être sous forme liquide ou solide, soit toutes préparations contentant lesdits extraits.

Dans un mode de réalisation avantageux de l'invention, le mélange préféré est le mélange de 3, 5-diCQ, 3, 4-diCQ et 4, 5-diCQ.

Les compositions utilisées dans le procédé selon l'invention comprennent au moins un acide dicaféoylquinique, ses analogues triacylés, ses analogues méthylés, l'acide 4-succinyl-3, 5-dicaféoylquinique ou un mélange de ceux-ci à une concentration comprise entre 0, 01 et 5 mM, avantageusement entre 0, 1 et 2 mM, plus avantageusement entre 0, 25 et 1mM.

Dans un mode de réalisation avantageux de l'invention, les pucerons sont choisis dans le groupe comprenant *Myzus persicae* (puceron vert du pêcher), *Myzus varians* (puceron cigarier du pêcher), *Myzus cerasi* (puceron noir du cerisier), *Brachycaudus persicae* (puceron noir du pêcher), *Aphis pomi* (puceron vert non migrant du pommier), *Brachycaudus helichrysi* (puceron vert du prunier), *Hyalopterus pruni* (puceron farineux du prunier), *Dysaphis plantaginea* (puceron cendré du pommier), *Dysaphis pyri,* puceron cendré (ou mauve) du poirier), *Aphis gossypii* (puceron du cotonnier ou du melon), *Acyrthosiphum pisum* (puceron du pois), *Macrosiphum euphorbiae* (puceron rose et vert de la pomme de terre), *Aphis spiraecola* (= *A. citricola)* (puceron vert des Citrus), *Aphis fabae* (puceron noir de la fève), *Rhopalosiphum maidis* (puceron vert du maïs), *Rhopalosiphum padi* (puceron du merisier à grappe), *Sitobion avenae* (Puceron des épis de céréales), *Diuraphis noxia* (puceron russe du blé), *Brevicoryne brassicae* (puceron cendré du chou), *Eriosoma lanigerum* (puceron lanigère du pommier), *Nasonovia ribisnigri* (puceron de la laitue), *Amphorophora idaei* (puceron du framboisier), *Toxoptera aurantii* (puceron noir des citrus et puceron du caféier), *Elatobium abietinum* (puceron vert de l'épicéa) et *Pemphigus bursarius* (puceron de la laitue).

Dans un mode de réalisation particulièrement avantageux de l'invention, le puceron est *Myzus persicae* (puceron vert du pêcher).

Les compositions utilisées dans le procédé selon l'invention sont appliquées sur les pucerons ou sur un endroit habité par lesdits pucerons.

La présente invention a également pour objet un procédé de lutte contre le puceron comprenant l'application d'au moins un composé de formule (I) ou leur mélange sur les pucerons ou sur un endroit habité par lesdits pucerons.

Les exemples 1 à 6 et les figures 1 à 24 qui suivent illustrent l'invention.

La figure 1 représente le chromatogramme CLHP à 330 nm d'un extrait d'apex de pêcher (5 CQ : acide 5-caféoylquinique ou acide chlorogénique, précurseur métabolique du 3, 5-diCQ). Les pics a, b et c représentent respectivement l'acide chlorogénique, le témoin interne et le 3, 5-diCQ. Chaîne d'analyse CLHP KONTRON : 2 pompes micro 420, injecteur-passeur d'échantillons 460, détecteur UV double longueur d'onde 430. Pilotage et acquisition par logiciel DIAMIR. Colonne MERCK LiChro CART 250-4- Superspher 100 RP18 endcapped, longueur 250 mm, diamètre 4 mm, placée dans un four thermostaté à 30°C. Phase mobile : solvant A : eau ultrapure acidifiée à pH 2, 6 avec de l'acide orthophosphorique, solvant B : méthanol qualité CLHP. Injection : 10μl d'extrait solubilisé dans du méthanol, filtré sur membrane PTFE 0, 45μm détection à 330 et 280 nm.

Le gradient d'élution est le suivant :

| | Temps (min) | Débit ml/min | % A | % B |
|---|---|---|---|---|
| Acquisition | 0,00 | 0,5 | 97 | 3 |
| | 5,00 | 0,5 | 97 | 3 |
| | 13,00 | 0,5 | 92 | 8 |
| | 23,00 | 0,5 | 88 | 12 |
| | 30,00 | 0,5 | 88 | 12 |
| | 33,00 | 0,5 | 87 | 13 |
| | 38,00 | 0,5 | 87 | 13 |
| | 40,00 | 0,5 | 86 | 14 |
| | 46,00 | 0,5 | 86 | 14 |
| | 50,00 | 0,5 | 84 | 16 |
| | 55,00 | 0,5 | 84 | 16 |
| | 57,00 | 0,5 | 83 | 17 |
| | 67,00 | 0,5 | 83 | 17 |
| | 82,00 | 0,5 | 81 | 19 |
| | 87,00 | 0,5 | 79 | 21 |
| | 95,00 | 0,5 | 79 | 21 |
| | 105,00 | 0,5 | 77 | 23 |
| | 107,00 | 0,5 | 77 | 23 |
| | 109,00 | 0,7 | 77 | 23 |
| | 115,00 | 0,7 | 77 | 23 |
| | 170,00 | 0,7 | 40 | 60 |
| | 171,00 | 0,7 | 40 | 60 |
| | 173,00 | 0,5 | 40 | 60 |
| | 175,00 | 0,5 | 40 | 60 |
| Rinçage | 180,00 | 0,5 | 97 | 3 |
| | 210,00 | 0,5 | 97 | 3 |

Dans ces conditions, le temps de rétention du 3, 5-diCQ est d'environ 142 min.

La figure 2 représente différents chromatogrammes CLHP de certains extraits.

La figure 2A représente le chromatogramme CLHP à 330 nm d'un extrait de racines d'*Ipomoea batatas* non tubérisées (5 CQ : acide 5-caféoylquinique où acide chlorogénique). Conditions CLHP sont identiques à celles de la figure 1. Les pics a, b et c représentent respectivement l'acide chlorogénique, le témoin interne et le 3, 5-diCQ.

La figure 2B représente le chromatogramme CLHP à 280 nm d'un extrait de racines d'*Ipomoea aquatica* non

tubérisées. Conditions CLHP sont identiques à celles de la figure 1. Les pics a, b, c et d représentent respectivement l'acide chlorogénique, le témoin interne, le 3, 4-diCQ et le 3, 5-diCQ.

La figure 2C représente le chromatogramme CLHP à 280 nm d'un extrait de racines d'*Ipomoea indica* non tubérisées. Conditions CLHP sont identiques à celles de la figure 1. Les pics a, b et c représentent respectivement l'acide chlorogénique, le témoin, interne et le 3, 5-diCQ.

La figure 2D représente le chromatogramme CLHP à 280 nm d'un extrait de racines de *Calystegia sepium* non tubérisées. Conditions CLHP sont identiques à celles de la figure 1. Les pics a, b, c, d et e représentent respectivement l'acide chlorogénique, le témoin interne, le 3, 4-diCQ, le 3, 5-diCQ et 4, 5-diCQ.

La figure 2E représente le chromatogramme CLHP à 280 nm d'un extrait de racines d'*Ipomoea nil* variété « Scarlett O'hara » non tubérisées. Conditions CLHP sont identiques à celles de la figure 1. Les pics a, b et c représentent respectivement l'acide chlorogénique, le témoin interne et le 3, 5-diCQ.

La figure 3 représente le chromatogramme CLHP à 330 nm du 3, 5-diCQ purifié de racines d'*Ipomoea batatas* non tubérisées. 3, 4-diCQ, a et b sont des isomères de 3, 5-diCQ. Chaîne d'analyse CLHP KONTRON : 2 pompes micro 420, injecteur-passeur d'échantillons 460, détecteur UV double longueur d'onde 430. Pilotage et acquisition par logiciel DIAMIR. Colonne MERCK LiChro CART 250-4 -Superspher 100 RP18 endcapped, longueur 250 mm, diamètre 4 mm, placée dans un four thermostaté à 30°C. Phase mobile: solvant A : eau ultrapure acidifiée à pH 2, 6 avec de l'acide orthophosphorique, solvant B : méthanol qualité CLHP. Injection : 10µl d'extrait solubilisé dans du Méthanol, filtré sur membrane PTFE 0.45µm, détection à 330 et 280 nm.

Gradient d'élution :

| | Temps (min) | Débit ml/min | %A | % B |
|---|---|---|---|---|
| Acquisition | 0,00 | 0,7 | 65 | 35 |
| | 1,00 | 0,7 | 65 | 35 |
| | 23,00 | 0,7 | 62 | 38 |
| | 30,00 | 0,7 | 62 | 38 |
| | 35,00 | 0,7 | 0 | 100 |
| | 40,00 | 0,7 | 0 | 100 |
| Equilibration | 41,00 | 0,7 | 65 | 35 |
| | 51,00 | 0,7 | 65 | 35 |

Dans ces conditions le 3, 5-diCQ est élué à un temps de 18 à 20 min.

La figure 4 illustre l'influence des conditions de culture sur la quantité de matière sèche racinaire produite (MS), la teneur en 3, 5-diCQ des racines et la quantité par bouture de 3, 5-diCQ produite dans les racines après 4 semaines de culture (moyenne de 5 boutures) (exemple 2, expérimentation A).

La figure 5 représente l'influence des conditions de culture sur (A) la quantité de matière sèche racinaire produite, (B) la teneur en 3, 5-diCQ des racines et (C) la quantité par bouture de 3, 5-diCQ racinaire produite après 4 semaines de culture (moyenne de 5 boutures). Les conditions de culture des boutures sont indiquées dans le tableau I de l'exemple 2 (expérimentation A).. Les barres verticales représentent l'écart-type. Les moyennes suivies d'une même lettre ne sont pas significativement différentes au seuil de 5% (test de Tukey).

La figure 6 illustre l'influence des conditions de carence minérale (azotée et phosphatée) sur la quantité de matière sèche racinaire produite, la teneur en 3, 5-diCQ des racines et la quantité par bouture de 3, 5-diCQ produite dans les racines après 3 semaines de culture (moyenne de 3 boutures) (exemple 2, expérimentation B).

La figure 7 représente l'influence des conditions de carence minérale (azotée et phosphatée) sur (A) la quantité de matière sèche racinaire produite, (B) la teneu. en 3, 5-diCQ des racines et (C) la quantité par bouture de 3, 5-diCQ racinaire produite après 3 semaines de culture (moyenne de 3 boutures) (expérimentation B). Les conditions de culture des boutures sont indiquées dans le tableau II de l'exemple 2. Les barres verticales représentent l'écart-type. Les moyennes suivies d'une même lettre ne sont pas significativement différentes au seuil de 5% (test de Tukey).

La figure 8 illustre la mise au point d'un système de production de racines par marcottage. Elle représente l'influence des milieux de bouturage et de marcottage sur la quantité de matière sèche produite, la teneur en 3, 5-diCQ et la quantité par plant de 3, 5-diCQ de racines issues de boutures (4 semaines de culture) et de marcottes (2 semaines de culture) (moyenne de 3 boutures).

La figure 9 illustre la mise au point d'un système de production de racines par marcottage. Elle représente l'influence des milieux de bouturage et de marcottage sur (A) la quantité de matière sèche produite, (B) la teneur en 3,5-diCQ et (C) la quantité par plant de 3, 5-diCQ de racines issues de boutures (4 semaines de culture) et de marcottes (2 semaines de culture) (moyenne de 3 boutures). Les conditions de culture des plants sont indiquées dans le tableau IV de l'exemple 3. Les milieux de culture sont l'eau distillée ou la solution minérale complète à 6 mM d'azote (composition dans le Tableau III de l'exemple 2). Les barres verticales représentent l'écart-type. Les moyennes suivies d'une même lettre ne sont pas significativement différentes au seuil de 5% (test de Tukey).

La figure 10 donne la composition du milieu de base dit Ap3 utilisé dans les exemples 2 et 3.

La figure 11 illustre le dispositif utilisé pour évaluer le comportement de larves de puceron en condition de choix entre deux milieux de composition différente (Chen, (1996), Thèse de l'Institut National des Sciences Appliquées, Lyon, France: 158 p).

La figure 12 illustre l'indice de phagostimulation vis-à-vis de *Myzus persicae,* de l'acide chlorogénique (A), de l'acide-para-coumarique (B), de l'acide caféique (C), de la cynarine (D), du méthyl ester de l'acide caféique (E), et de l'acide 3, 5-dicaféoylquinique (F) dissous à différentes concentrations dans le milieu de référence Ap3 et testés contre le milieu Ap3 (tests de choix). L'effet répulsif (indice de phagostimulation négatif) est significatif pour les points expérimentaux accompagnés d'un astérisque (P < 0, 05), de deux (*P* < 0, 01) ou de trois (P < 0, 001) (test des rangs de Wilcoxon). La dose 0 mM correspond au milieu témoin Ap3.

La figure 13 illustre l'indice de phagostimulation de l'acide 3, 5-dicaféoylquinique (3, 5-diCQ) et de son précurseur l'acide chlorogénique (AC) vis-à-vis de *Myzus persicae,* testés contre le milieu témoin Ap3 ou l'un contre l'autre. L'effet répulsif (indice négatif) est significatif pour les points expérimentaux accompagnés d'un astérisque (*P* < 0, 05), de deux (*P* < 0, 01) ou de trois (*P* < 0, 001) (test des rangs de Wilcoxon). La concentration 0 mM (carré noir) correspond au milieu témoin Ap3.

La figure 14 illustre l'indice de phagostimulation de l'acide 3, 5-dicaféoylquinique (3, 5-DiCQ) à 0, 5 mM contre des molarités décroissantes de son précurseur l'acide chlorogénique (AC) vis-à-vis de *Myzus persicae.* L'effet répulsif (indice négatif) est significatif pour les points expérimentaux accompagnés d'un astérisque (*P* < 0, 05), de deux (*P* < 0, 01) ou de trois (*P* < 0, 001) (test des rangs de Wilcoxon).

La figure 15 illustre l'effet phagorépulsif sur *M. persicae* de l'acide 3, 5-dicaféoylquinique (3, 5-diCQ) (A), de l'isomère 4, 5-diCQ (B) et du mélange 3, 4-diCQ/3, 5-diCQ/4, 5-diCQ (C) par rapport au milieu témoin Ap3. Pour le mélange d'isomères, la dose indique la teneur totale en diCQ. L'effet répulsif (indice de phagostimulation négatif) est significatif pour les points expérimentaux accompagnés d'un astérisque (*P* < 0, 05), de deux (*P* < 0, 01) ou de trois (P < 0, 001). La dose 0 correspond au milieu Ap3.

La figure 16 illustre la mortalité larvaire cumulée de *Myzus persicae* obtenue au cours des tests d'évaluation de la toxicité de l'acide 3, 5-dicaféoylquinique (3, 5-diCQ), de son précurseur l'acide chlorogénique (AC); du milieu témoin Ap3 et de l'extrait témoin obtenu à partir des solvants et additifs utilisés pour la purification du 3, 5-diCQ. Le milieu Ap3 est ajusté à pH 7, 5.

La figure 17 illustre le poids des adultes aptères (moyenne + 1 écart-type) de *Myzus persicae* élevés sur milieu témoin Ap3, sur l'extrait témoin obtenu à partir des solvants et additifs utilisés pour la purification du 3, 5-diCQ, et sur l'acide 3, 5-dicaféoylquinique (3, 5-diCQ). Les moyennes suivies d'une même lettre ne sont pas significativement différentes au seuil de 5% (test de Student-Newman-Keuls).

La figure 18 illustre le poids des adultes aptères (moyenne + 1 écart-type) de *Myzus persicae* élevés sur milieu témoin Ap3 et sur l'acide chlorogénique. Les moyennes suivies d'une même lettre ne sont pas significativement différentes au seuil de 5% (test de Student-Newman-Keuls).

La figure 19 illustre la mortalité journalière cumulée observée au cours du développement larvaire de *Myzus persicae* (7 jours) en présence d'acide 3, 5-dicaféoylquinique, de deux de ses isomères (4, 5-diCQ, 1, 5-diCQ) et du mélange 3, 4-diCQ/3, 5-diCQ/4, 5-diCQ. La gamme de concentration de la substance testée varie 0, 03125 mM à 1 mM. La dose 0 correspond au milieu témoin Ap3 seul.

La figure 20 illustre le poids moyen (moyenne ± 1 écart-type) d'un puceron parvenu à l'état adulte aptère au terme du développement larvaire sur milieu nutritif Ap3 supplémenté ou non par l'acide 3-5 dicaféoylquinique, ses isomères (4, 5-diCQ, 1, 5-diCQ), et le mélange 3, 4-diCQ/3, 5-diCQ/4, 5-diCQ. Les moyennes suivies d'une même lettre ne sont pas significativement différentes au seuil de 5% (test de Bonferroni/Dunn).

La figure 21 illustre l'effet phagorépulsif de l'acide 3, 5-dicaféoylquinique vis-à-vis d'*Acyrtosiphon pisum.* L'effet répulsif (indice de phagostimulation négatif) est significatif pour les points expérimentaux accompagnés d'un astérisque (P < 0, 05), de deux (P < 0, 01) ou de trois (P < 0, 001). La dose 0 correspond au milieu témoin Ap3.

La figure 22 illustre la mortalité journalière cumulée observée au cours du développement larvaire d'*Acyrtosiphon pisum* en présence d'acide 3, 5-dicaféoylquinique. La gamme de concentration en 3, 5-diCQ varie 0, 03125 mM à 1 mM. La dose 0 correspond au milieu témoin Ap3 seul.

La figure 23 illustre l'effet phagorépulsif de l'acide 3, 5-dicaféoylquinique vis-à-vis de *Macrosiphum euphorbiae.* L'effet répulsif (indice de phagostimulation négatif) est significatif pour les points expérimentaux accompagnés d'un astérisque (*P* < 0, 05), de deux (*P* < 0, 01) ou de trois (P < 0, 001). La dose 0. correspond au milieu témoin Ap3.

La figure 24 illustre la mortalité journalière cumulée observée au cours du développement larvaire de *M.*

*euphorbiae* en présence d'acide 3, 5-dicaféoylquinique. La gamme de concentration en 3, 5-diCQ varie 0, 03125 mM à 1 mM. La dose 0 correspond au milieu témoin Ap3 seul.

**Exemple 1 : Extraction de l'acide 3,5-diCQ de la racine non tubérisée d'*Ipomoea batatas***

Des tubercules de patate douce (*Ipomoea batatas*) achetés dans le commerce ont été mis en culture. Le support de culture est constitué d'un récipient d'une contenance de 1, 5 L environ, protégé de la lumière. Les récipients sont remplis d'eau distillée et les tubercules y sont immergés à 80 %. La culture est réalisée au laboratoire en lumière naturelle.

Les racines non tubérisées formées par les tubercules sont prélevées environ 7 mois après la mise en culture, immédiatement congelées dans l'azote liquide et lyophilisées. Les racines sont ensuite broyées dans un mortier refroidi par de l'azote liquide puis de nouveau lyophilisées pour obtenir une poudre sèche. Cette poudre est conservée à- 20 °C.

On extrait 10 g de poudre sèche à trois reprises avec de l'éthanol à 70 % avec à chaque extraction homogénéisation à l'ultra-turrax de la poudre ou du résidu dans environ 80 mL de solvant, puis agitation 15 min en chambre froide (4°C) et filtration sur verre fritté. Après la dernière extraction, on rince le résidu par 60 mL d'éthanol à 70%. Le volume total de l'extrait hydroalcoolique est donc d'environ 300 mL. On évapore l'éthanol à l'aide d'un évaporateur rotatif pour obtenir une phase aqueuse de 10 à 20 mL complétée par de l'eau distillée jusqu'à un volume de 50 mL. On extrait cette phase aqueuse par 100 mL d'acétate d'éthyle après adjonction de chlorure de sodium (8%, Poids/Volume final phase aqueuse = 100 mL) et d'acide métaphosphorique (1 %, Poids/Volume final phase aqueuse). On réalise 4 extractions successives, on sèche les traces d'eau résiduelles par adjonction dans la phase organique de quelques mg de sulfate de sodium anhydre. Après filtration sur coton de verre on évapore à sec la phase organique dans un évaporateur rotatif et on reprend le culot par 8 mL de méthanol qualité CLHP. On filtre sur membrane PTFE 0, 45μm. On sépare le 3, 5-diCQ par 15 chromatographies successives sur une chaîne CLHP semi-préparative WATERS® 600 (solvants: eau ultra pure acidifiée à pH 2, 6 par l'acide o-phosphorique (solvant A) et méthanol CLHP (solvant B)) sur une colonne INTERCHIM (longueur 250 mm ; diamètre 21, 2 mm) remplie d'une phase stationnaire C18 Uptisphère 10 μm et thermostatée à 30 °C. Le débit du système est de 18 mL/min. Le volume injecté est de 500 μL. Le gradient utilisé est le suivant : 0 min : 35 % B ; 10 min : 35 % B ; 23 min : 38 % B ; 33 min : 38 % B. On recueille le 3, 5-diCQ dans la phase mobile environ 25 min après l'injection. On réduit les fractions recueillies par concentration à l'aide d'un évaporateur rotatif jusqu'à obtention d'une phase aqueuse de 70 mL environ. On ajuste à 100 mL avec de l'eau distillée et on extrait 3 fois avec 200 mL d'acétate d'éthyle après addition de chlorure de sodium (10% Poids/Volume final phase aqueuse = 200 mL). On sèche la phase organique par adjonction de quelques mg de sulfate de sodium anhydre. Après filtration sur coton de verre, on évapore l'acétate d'éthyle à sec puis on reprend le résidu dans 10 mL de méthanol. On filtre sur membrane PTFE 0, 45 μm. On ajoute 30 mL d'eau ultra pure froide pour faire précipiter la molécule, on congèle dans l'azote liquide et on lyophilise.

On obtient 91, 9 mg de poudre blanche anhydre contenant environ 93 % de 3, 5-diCQ et 7 % de formes isomères (figure 3, chromatogramme de contrôle de pureté de la substance obtenu sur chaîne CLHP analytique).

**Exemple 2: Conditions de culture favorables à la production d'acides dicaféoylquiniques dans les racines d'Ipomoea batatas**

**2.1. Matériel et méthode**

Deux expérimentations successives (A et B) ont été menées sur une variété de la patate douce à chair rouge en provenance de Guadeloupe (clone IbD) cultivée en conteneurs de 7 L sous tunnel isolé des insectes à Avignon. Des boutures de 40 à 50 cm de longueur, constituées par l'extrémité des tiges en croissance ont été prélevées, effeuillées sur la partie basale pour éviter le contact des feuilles avec le milieu de culture, et placées dans des récipients en plastique transparent, d'une contenance de 1, 25 L environ, remplis par les différents milieux et substrats de culture testés, et protégés ou non de la lumière par une feuille d'aluminium. Les récipients contenant un substrat solide sont percés à la base afin de permettre le drainage du milieu apporté. Les racines se développant au niveau des noeuds de la tige, un nombre égal de noeuds de la base de la bouture a été placé dans le milieu de culture.

L'expérimentation A a permis de tester l'influence de trois facteurs différents sur la production de racines et leur teneur en 3, 5-diCQ : la nature du substrat de culture, aéré ou non (perlite ou milieu liquide), l'effet de la lumière (culture des racines à l'obscurité ou à la lumière), et l'influence de la composition minérale du milieu de culture (milieu complet, milieu carencé en azote ou eau distillée). Les différentes conditions de culture comparées dans l'expérimentation A sont illustrées dans le tableau I.

TABLEAU I

| Condition | Milieu de culture | Apports nutritifs | Apport d'azote | Eclairement racines |
|---|---|---|---|---|
| A | liquide | Eau distillée | 0 mM | Obscurité |
| B | liquide | Eau distillée | 0 mM | Lumière |
| C | liquide | Solution nutritive | 0,05 mM | Obscurité |
| D | liquide | Solution nutritive | 6 mM | Obscurité |
| E | perlite | Eau distillée | 0 mM | Obscurité |
| F | perlite | Solution nutritive | 6 mM | Obscurité |

L'expérimentation B a permis de comparer l'influence respective des carences totales en azote et en phosphate sur la production de racines et leur teneur en 3, 5-diCQ. Les différentes conditions de cultures comparées dans l'expérimentation B sont illustrées dans le tableau II.

TABLEAU II

| **Condition** | **Milieu de culture** | **Apports nutritifs** | **Apport d'azote** | **Apport de phosphore** | **Eclairement des racines** |
|---|---|---|---|---|---|
| E | liquide | Eau distillée | 0 mM | 0 mM | Obscurité |
| F | liquide | Solution nutritive | 6 mM | 1 mM | Obscurité |
| G | liquide | Solution nutritive | 0 mM | 1 mM | Obscurité |
| H | liquide | Solution nutritive | 6 mM | 0 mM | Obscurité |

5 et 3 répétitions par condition ont été respectivement mises en place pour les expérimentations A et B. Les plantes disposées de manière randomisée ont été cultivées dans une enceinte climatisée à une température de 25°C et sous une photopériode de 16 h de jour et 8 h de nuit. Le volume de milieu liquide est complété régulièrement au niveau initial et les plantes cultivées sur substrat solide arrosées fréquemment avec la solution de culture. Les racines formées ont été prélevées au bout de quatre semaines (expérimentation A) ou trois semaines (expérimentation B) de culture, pesées, placées dans l'azote liquide, lyophilisées, broyées dans l'azote liquide puis lyophilisées de nouveau. Les poudres obtenues ont été analysées par CLHP pour leur contenu en composés phénoliques après extraction dans le solvant éthanol/eau (70/30, v à v), évaporation à sec, reprise de l'extrait sec dans le méthanol et filtration.

Dans les deux expérimentations, la solution nutritive apportée est une solution nutritive complète comprenant macro et micro-éléments dont la composition est donnée en tableau III. Les teneurs en azote, apporté exclusivement sous forme nitrate, et en phosphate varient selon les situations de carence étudiées.

TABLEAU III

| **en mg (ou μL) par L** | **Solutions** | | | |
|---|---|---|---|---|
| **Produit** | **6 mM N*** | **0,05 mM N** | **0 N** | **0 P** |
| $K_2SO_4$ | 174 | 174 | 174 | 261 |
| $Ca(NO_3)_2$, $4H_2O$ | 708 | 6 | 0 | 708 |
| $MgSO_4$, $7H_2O$ | 370 | 370 | 370 | 370 |
| $CaSO_4$, $2H_2O$ | 86 | 598 | 603 | 86 |
| EDTA-Fe, $1H_2O$ | 17 | 17 | 17 | 17 |
| $KH_2PO_4$ | 136 | 136 | 136 | 0 |
| Kanieltra 6 Fe (μL) | 100 | 100 | 100 | 100 |

**2.2. Résultats**

Ils sont donnés dans les figures 4 à 7.

Les résultats de l'expérimentation A (figures 4 et 5) montrent que les conditions de culture exercent une forte influence sur la teneur en 3, 5-diCQ des racines.

Les teneurs maximales en 3, 5-diCQ sont observées dans les racines produites dans l'eau distillée et à la lumière (condition B) où elles dépassent 9 % de la matière sèche. Les teneurs sont minimales dans les racines produites sur substrat aéré de type perlite avec une solution minérale complète (condition F). Elles restent cependant élevées, avec environ 1, 5 % de la matière sèche, dans ces conditions de culture comportant une solution minérale contenant de l'azote uniquement sous forme de nitrate. Les teneurs obtenues dans les autres conditions de culture montrent que la production de racines en milieu liquide et en situation de carence azotée tend à augmenter les teneurs en 3, 5-diCQ (figures 4 et 5B).

La quantité de racines produites à partir des boutures est également fortement affectée par les conditions de cultures. La quantité de matière sèche racinaire dans les conditions de fertilisation complète (conditions D et F) est très supérieure à celle produite dans les autres conditions (figures 4 et 5A). Ceci conduit à une quantité totale de 3, 5-diCQ produite par bouture équivalente dans les conditions permettant une forte teneur de 3, 5-diCQ dans les racines mais pénalisant leur croissance (condition A) et dans les conditions donnant une teneur plus faible en 3, 5-diCQ mais permettant une croissance racinaire forte (conditions D et F) (figures 4 et 5C).

Les résultats de l'expérimentation B (figures 6 et 7) montrent que les conditions de carence minérale, azotée ou phosphatée, exercent une influence sur la teneur en 3, 5-diCQ des racines.

Comme dans l'expérimentation A, les teneurs les plus fortes en 3, 5-diCQ sont obtenues en culture dans l'eau distillée et les plus faibles dans la solution minérale complète. En culture dans des solutions minérales carencées en azote ou en phosphate les teneurs en 3, 5-diCQ sont intermédiaires (figures 6 et 7B). Ceci montre que les conditions de carences en phosphate et en azote tendent l'une et l'autre à augmenter la teneur en 3, 5-diCQ des racines par rapport à la condition de fertilisation complète mais de façon moins importante que la situation de carence totale en éléments minéraux, dans l'eau distillée. A l'inverse, ces conditions de carences azotée et phosphatée limitent la croissance racinaire par rapport à la solution minérale complète, de manière cependant moindre que dans l'eau distillée (figures 6 et 7A). Il en résulte que la quantité de 3, 5-diCQ produit dans les racines par bouture est équivalente pour toutes les conditions (figures 6 et 7C).

### 2.3. Conclusion

Les conditions de culture influent fortement sur la teneur en 3, 5-diCQ des racines non tubérisées de la patate douce *Ipomoea batatas.* La culture en milieu liquide, à la lumière et en situation de carence minérale, notamment azotée et phosphatée, sont des facteurs favorisant l'accumulation de l'acide 3, 5-dicaféoylquinique dans les racines produites par des boutures. Les teneurs maximales, supérieures à 90 $mg.g^{-1}$ de matière sèche, ont été obtenues dans des racines produites dans l'eau et à la lumière. Ces conditions de culture limitent cependant fortement la production des racines obtenues par bouturage de tige. La culture des boutures dans une solution nutritive complète contenant de l'azote sous forme de nitrate uniquement, permet une production de racines plus importante présentant une teneur en 3, 5-diCQ relativement élevée, variant de 20 à 25 $mg.g^{-1}$ de matière sèche.

## Exemple 3 : Mise au point d'un système de production de racines par marcottage

### 3.1. Matériel et méthodes

Des tiges dont l'extrémité est en croissance, d'environ 110 cm de longueur, ont été prélevées sur le clone IbD de patate douce. Leur base a été défeuillée sur quatre noeuds, soit une longueur de 20 cm environ, et a été mise en bouturage en chambre climatisée dans des récipients de 1.25 L environ, contenant de l'eau distillée ou une solution minérale complète et protégés de la lumière par une feuille d'aluminium. La composition des solutions nutritives utilisées pour la production par bouturage de racines de patate douce *Ipomoea batatas* sont celles illustrées dans le tableau III. Les plantes ont été cultivées dans une enceinte climatisée à une température de 25°C et sous une photopériode de 16 h de jour et 8 h de nuit. Le volume de milieu liquide a régulièrement été complété au niveau initial. Après 15 jours de culture, les plants sont enracinés et servent de plantes mères à la production des marcottes. Pour cela, sur chaque plant une portion de tige d'une dizaine de noeuds a été défeuillée et mise en marcottage par immersion dans ces bacs de 20 L environ, remplis d'eau ou de solution minérale et abrités de la lumière par une feuille d'aluminium.

Quatre conditions expérimentales ont été mises en place faisant varier le milieu de bouturage ou de marcottage, eau ou solution minérale complète. Ces conditions expérimentales sont illustrées dans le tableau IV.

TABLEAU IV

| Condition | Milieu bouturage (4 semaines de culture) | Milieu marcottage (2 semaines de culture) |
|---|---|---|
| A | Eau distillée | Eau distillée |
| B | Eau distillée | Milieu nutritif 6 mM d'azote |
| C | Milieu nutritif 6 mM d'azote | Milieu nutritif 6 mM d'azote |
| D | Milieu nutritif 6 mM d'azote | Eau distillée |

3 plants ont été cultivés par condition. Les racines ont été prélevées sur la partie bouturée et sur la partie marcottée de chaque plante 15 jours après la mise en marcottage des tiges soit 4 semaines après la mise en bouturage.

Les racines formées ont été prélevées, pesées, placées dans l'azote liquide, lyophilisées, broyées dans l'azote liquide puis lyophilisées de nouveau. Les poudres obtenues ont été analysées par CLHP pour leur contenu en composés phénoliques après extraction dans le solvant éthanol/eau (70/30, v à v), évaporation à sec, reprise de l'extrait sec dans le méthanol et filtration.

### 3.2. Résultats

Ils sont donnés dans les figures 8 et 9.

Les résultats montrent clairement que la teneur en 3, 5-diCQ dépend du milieu de culture utilisé localement pour la production des racines, que ce soit par bouturage ou marcottage, et n'est pas influencée par la nature du milieu apporté pour la production du système racinaire distant (figures 8 et 9B).

Les teneurs moyennes en 3, 5-diCQ des racines produites dans l'eau varient de 44.7 à 47, 4 $mg.g^{-1}$ de matière sèche dans les racines de boutures et de 31, 9 à 35, 5 $mg.g^{-1}$ de matière sèche dans les racines de marcottes. Les teneurs moyennes en 3, 5-diCQ des racines produites dans la solution minérale sont trois fois plus faibles. Elles varient de 15, 5 à 16, 2 $mg.g^{-1}$ de matière sèche dans les racines de boutures et de 10, 2 à 11, 6 $mg.g^{-1}$ de matière sèche dans les racines de marcottes.

La nature du milieu apporté localement influence la quantité de matière sèche produite (figure 8A). Cependant, de manière inattendue, elle semble également être influencée par le milieu de culture utilisé pour la production du milieu de culture distant. En effet la quantité moyenne de racines issues de boutures produites dans la solution minérale (condition D) est significativement supérieure quand les marcottes sont effectuées dans l'eau à celle des boutures dont la partie marcottée est dans la solution minérale (condition C). Les quantités moyennes totales de 3, 5-diCQ produit dans les racines formées par bouturage (31, 1 $mg.g^{-1}$ de matière sèche), par marcottage (26, 2 $mg.g^{-1}$ de matière sèche) et leur somme (57, 3 $mg.g^{-1}$ de matière sèche) sont les plus élevées dans cette condition D (bouturage dans la solution minérale et marcottage dans l'eau).

### 3.3. Conclusion

La teneur en 3, 5-diCQ des racines obtenues par marcotte dépend du milieu de culture utilisé localement pour leur production et n'est pas influencée par les conditions de culture de la plante mère. Le marcottage dans l'eau de plants de patate douce normalement fertilisés permet la production de racines présentant des teneurs élevées en 3, 5-diCQ. Ce système permet de concilier une croissance importante de la plante-mère par une alimentation minérale appropriée et la production de racines issues de marcottes présentant des teneurs élevées en 3, 5-diCQ. L'intérêt de ce système a été validé en culture en extérieur sur deux clones de patate douce, cultivées en container sous tunnel et normalement fertilisés. Les tiges mises en marcottage dans l'eau ont produit en 15 jours une masse racinaire importante présentant des teneurs élevées en 3, 5-diCQ (Clone IbD: 32, 7 $mg.g^{-1}$ matière sèche ; Clone IbE: 40, 4 $mg.g^{-1}$ matière sèche).

## Exemple 4 : Mesure de la répulsivité du 3,5-diCQ, de ses isomères et de leur mélange vis-à-vis du puceron vert du pêcher *(Myzus persicne)*

### 4.1. Mode opératoire

La méthode utilisée est celle développée par Yvan Rahbé (Rahbé & Febvay (1993) Entomologia Experimentalis et Applicata 67: 149-160 ; Chen (1996) Thèse de l'Institut National des Sciences Appliquées, Lyon, France: 158 p) dans le cadre de recherches sur les déterminants chimiques de la résistance des plantes à diverses espèces de pucerons *(Aphis gossypii, Acyrthosiphon pisum),* et adaptée au puceron vert *Myzus persicae.*

L'objectif est d'évaluer le caractère attractif ou répulsif d'une molécule candidate, à différentes concentrations, soit par rapport à un milieu de base témoin, soit par rapport à une autre substance. Le milieu de base utilisé, dit Ap3 (composition dans le tableau de la figure 10), a été mis au point à l'UMR INRA-INSA de Lyon pour permettre un développement optimal de l'espèce de puceron *Acyrtosiphon pisum* (Febvay et al. (1988) Canadian Journal of Zoology 66: 2149-2453). Il permet aussi le développement de *Myzus persicae.* Ce milieu de base est supplémenté ou non par les différents acides phénoliques testés. Le comportement de choix du puceron, mis en présence des deux milieux, est étudié dans un système clos où l'insecte n'a pas d'autre alternative pour se nourrir que de choisir l'un des deux milieux. Le dispositif est schématisé dans la Figure 11.

Le milieu (35 µl) est coulé entre deux membranes de Parafilm® étirées sur un cône Eppendorf®. Sur un portoir comportant 6 cages expérimentales, un milieu témoin est disposé en face d'un milieu testé sur chaque cage. Les répétitions d'une même modalité (milieu témoin ou milieu testé) sont disposées en alternance le long du portoir, pour éviter tout biais systématique lors du dépôt des pucerons. Six larves de puceron (second ou troisième stade) sont déposées en fin de journée dans chacune des cages d'un portoir. Les portoirs sont ensuite introduits dans une boîte noire placée dans une enceinte climatique à 19°C pendant 15 heures. Ce passage à l'obscurité permet de stabiliser plus rapidement la fixation des larves. Le lendemain matin, le nombre de pucerons fixés sur chaque milieu est alors noté. Les tests sont effectués en routine sur 24 répétitions de choix (4 portoirs comportant chacun 6 cages). Un indice de phagostimulation, représentant le degré d'appétence pour le puceron du milieu testé, est calculé de la façon suivante :

**Indice de phagostimulation = (Nb test – Nb témoin) / Nb total,**

où :

Nb test = Nombre de pucerons fixés sur le milieu testé,
Nb témoin = Nombre de pucerons fixés sur le milieu témoin,
Nb total = Nombre total de pucerons fixés.

Par construction, la valeur de l'indice est comprise entre -1 et 1. Un indice négatif signifie que le milieu testé est répulsif par rapport au milieu témoin, et un indice positif signifie que le milieu testé est attractif. L'hypothèse que l'effet du milieu testé est identique à celui du milieu témoin est examinée par le test des rangs de Wilcoxon.

Les substances phénoliques testées autres que le 3, 5-diCQ ont une origine commerciale excepté le méthyl ester de l'acide caféique qui a été synthétisé. Les molécules ont été caractérisées par spectrométrie de masse et résonance magnétique nucléaire du proton. La teneur et la stabilité des molécules dissoutes sont contrôlées par CLHP après dissolution des molécules dans le milieu Ap3, au début et en fin d'expérimentation.

### 4.2. Résultats

#### 4.2.1 Effet répulsif du 3,5-diCQ

Ils sont donnés dans les figures 12 à 14.

Les résultats obtenus mettent en évidence un effet répulsif significatif de l'ensemble des dérivés caféiques.

Le 3, 5-diCQ et le méthyl ester de l'acide caféique sont les deux composés les plus répulsifs : ils ont un effet hautement significatif dès la plus faible concentration (0, 25 mM), alors que les effets de l'acide chlorogénique et de la cynarine ne sont significatifs qu'à partir des concentrations 0, 5 mM et 1 mM respectivement. L'acide para-coumarique, qui est une molécule phénolique mono hydroxylée, n'a aucun effet répulsif sur *Myzus persicae* dans la gamme de concentration testée (figure 12). On a également vérifié qu'une solution témoin réalisée avec les solvants et additifs utilisés pour l'extraction et la purification du 3, 5-diCQ ne montre un effet répulsif qu'à une concentration largement supérieure à celle utilisée lors de la préparation de la molécule (données non montrées).

Le 3, 5-diCQ est significativement répulsif dès la concentration de 0, 125 mM et l'acide chlorogénique (AC) à 0, 25 mM (figure 13). Pour une même molarité, le puceron choisi systématiquement AC plutôt que le 3, 5-diCQ dès 0, 125 mM. On a obtenu des résultats identiques en utilisant un milieu Ap3 à pH 5, 3 dans lequel les substances testées ne montrent aucun brunissement, contrairement au milieu Ap3 originel ajusté à pH 7, 5 dans lequel les substances caféiques montrent un brunissement important (résultats non montrés).

Lorsqu'on fait varier la concentration d'AC de 0, 5 mM à 2 mM face à 3, 5-diCQ 0, 5mM, les résultats obtenus montrent que le 3, 5-diCQ à une molarité de 0, 5 mM reste répulsif par rapport à l'acide chlorogénique à une concentration quatre fois supérieure (2 mM) pour un pH de 7, 5 (Figure 14). Des résultats similaires ont été obtenus à pH 5, 3.

L'ensemble des résultats obtenus montre un net effet répulsif de l'acide 3, 5-dicaféoylquinique vis-à-vis du

puceron vert du pêcher (*Myzus persicae*). Cet effet est détectable à une concentration minimale de 125 µM (soit 64, 5 mg.$L^{-1}$). L'effet répulsif de cette molécule est nettement plus fort que celui de son précurseur métabolique, l'acide chlorogénique.

#### 4.2.2 Effet répulsif des isomères du 3,5-diCQ et de leur mélange sur *Myzus persicae*

Les résultats sont donnés dans la figure 15.

Les effets répulsifs du 3, 5-diCQ, du 4, 5-diCQ et du mélange 3, 4-diCQ/3, 5-diCQ/4, 5-diCQ (en proportions égales) ont été testés aux mêmes concentrations. La gamme de concentration de la substance testée ou du mélange varie de 0, 03125 à 1 mM.

Le 3, 5-diCQ et de l'ensemble de ses isomères représentent un effet répulsif significatif. Le 3, 5-diCQ, le 4, 5-diCQ et le mélange 3, 4-diCQ/3, 5-diCQ/4, 5-diCQ ont un effet répulsif hautement significatif dès la dose 0, 125 mM. Des effets répulsifs significatifs ont pu être détectés dès la dose de 0, 03125 mM pour le 4, 5-diCQ et le mélange 3, 4-diCQ/3, 5-diCQ/4, 5-diCQ.

L'acide 3, 5-dicaféoylquinique, l'acide 4, 5-dicaféoylquinique et le mélange 3, 4-diCQ/3, 5-diCQ/4, 5-diCQ présentent tous une activité répulsive sur *Myzus persicae.* Cette activité varie cependant en fonction de la molécule présentée au puceron. Cela suggère une influence de la position d'acylation des acides caféiques sur l'acide quinique sur le caractère répulsif des acides dicaféoylquiniques.

## Exemple 5 : Mesure de la toxicité du 3,5-diCQ, de ses isomères et de leur mélange sur le développement larvaire du puceron vert du pêcher (*Myzus persicae*)

### 5.1. Mode opératoire

L'objectif est de quantifier l'effet toxique du 3, 5-diCQ et ses dérivés sur le puceron pendant la durée de son développement larvaire, soit 8 à 10 jours pour *Myzus persicae* à 19° C. La molécule est ajoutée à différentes concentrations au milieu nutritif Ap3.

Le milieu (75 µl) est coulé entre deux membranes de Parafilm® stérilisées aux UV et étirées sur un support cylindrique en PVC (h = 1, 5 cm, d = 2 cm), en conditions stériles. Le Jour J0, 20 larves néonates sont déposées à l'intérieur du cylindre, lequel est ensuite retourné et appliqué sur un carré de Parafilm®. Les boîtes sont placées sous lumière tamisée (16h jour/8h nuit) et dans une enceinte à 19° C. Le pourcentage de fixation est noté après 1 heure comme indicateur de phagostimulation à court terme et pour vérifier qu'aucune anomalie ne s'est produite au moment du dépôt des pucerons. La mortalité larvaire est relevée quotidiennement en fin de journée. Le jour J+3, les larves sont prélevées et dédoublées dans deux nouvelles boîtes. Le jour de leur mue imaginale, les pucerons ayant atteint le stade adulte aptère sont déposés successivement sur une balance de précision (d = 0, 01 mg) et les poids cumulés sont notés pour chaque concentration de chaque molécule testée. Les poids individuels sont calculés ultérieurement. Les effets d'une même molécule à toutes les concentrations et les effets du milieu témoin sont évalués simultanément pendant le même test. Les tests sont effectués en routine sur 3 répétitions.

### 5.2. Résultats

#### 5.2.1 Effet toxique du 3,5-diCQ sur *Myzus persicae*

*5.2.1.1. Effet sur la mortalité larvaire*

Les résultats sont donnés dans la figure 16.

On observe un effet très marqué du 3, 5-diCQ sur la mortalité larvaire du puceron, qui augmente progressivement au cours du développement de l'insecte. La mortalité augmente également avec la concentration, pour atteindre pratiquement 100% à 2 mM. En revanche, la mortalité n'excède jamais 10% sur le milieu Ap3, sur l'extrait témoin obtenu à partir des solvants et additifs utilisés pour la purification du 3, 5-diCQ, et sur l'acide chlorogénique (y compris aux concentrations les plus élevées).

*5.2.1.2. Effet sur le poids des adultes aptères*

Les résultats sont donnés aux figures 17 et 18.

Les pesées des adultes aptères survivants obtenus au terme de leur développement mettent en évidence un effet dépressif significatif du 3, 5-diCQ à partir de la dose 0, 25 mM (Figure 17) (ANOVA : $F$= 32, 86, $P$ < 0, 0001). Les concentrations 1 et 2 mM du 3, 5-diCQ ne sont pas représentées car aucun puceron n'a atteint le stade adulte aptère

sur ces modalités. Cet effet dépressif sur le poids des pucerons n'est pas observé pour l'acide chlorogénique, qui semble exercer plutôt un effet probiotique (Figure 18; ANOVA : *F* = 3, 31, *P* = 0, 0063).

Les tests *in vitro* montrent que le 3, 5-diCQ provoque une forte mortalité des larves pendant leur développement et diminue significativement le poids des adultes obtenus, alors que le précurseur métabolique AC ne montre aucun effet négatif sur le développement larvaire.

### 5.2.2 Effet toxique des isomères du 3,5-diCQ et de leur mélange sur *Myzus persicae*

*5.2.2.1. Effet sur la mortalité larvaire*

Les résultats sont donnés dans la figure 19.

On observe un effet toxique très marqué de tous les isomères testés et du mélange d'isomères à la dose 1 mM (80 à 100% de mortalité cumulée observée dès le $3^{ème}$ jour de développement larvaire). Le 4, 5-diCQ et le 1, 5-diCQ montrent un effet toxique net à la dose 0, 5 mM (mortalité cumulée supérieure à 50 % au $3^{ème}$ jour). Le mélange d'isomères provoque une mortalité cumulée supérieure à 50 % au $3^{ème}$ jour dès la dose 0, 25 mM.

*5.2.2. 2. Effet sur le poids des adultes aptères*

Les résultats sont donnés dans la figure 20.

Les pesées des adultes aptères obtenus au terme de leur développement (Figure 20) mettent en évidence un effet dépressif significatif à la dose 0, 5 mM pour le 3, 5-diCQ (ANOVA : F =10, 856, P < 0, 0001). Cet effet dépressif sur le poids des pucerons est observé dès la dose 0, 25 mM pour le 4, 5-diCQ (ANOVA : F =38, 61, P < 0.0001) et le mélange d'isomères (ANOVA : F = 39, 248, P < 0.0001). En revanche aucun effet sur le poids des pucerons n'est observé pour la cynarine à la dose 0, 25 mM (ANOVA : F = 2, 291, P = 0, 068).

L'acide 3, 5-dicaféoylquinique, l'acide 4, 5-dicaféoylquinique, l'acide 1, 5-dicaféoylquinique et le mélange des acides 3, 4-dicaféoylquinique/3, 5-dicaféoylquinique/4, 5-dicaféoylquinique présentent tous une activité toxique sur *Myzus persicae.*

**Exemple** 6 : **Evaluation *in vitro de* l'effet de l'acide 3,5-dicaféoylquinique sur d'autrés espèces de pucerons**

### 6.1. Matériel animal et méthodes

Deux espèces de pucerons, dont le puceron vert ou rose du pois, *Acyrthosiphon pisum* et le puceron vert et rose de la pomme de terre, *Macrosiphum euphorbiae,* sont utilisées dans cette étude.

*Acyrthosiphon pisum,* (Harris) est un puceron de grande taille qui colonise les légumineuses sauvages et cultivées telles que le Sainfoin, la Luzerne, le Lotier, la Vesce, les Pois, le Haricot et le Trèfle. Le clone de puceron fourni par l'UMR BF2I INRA/INSA de Lyon est élevé en chambre climatisée sur fève.

*Macrosiphum euphorbiae* (Thomas) est un puceron de grande taille et fusiforme. Très polyphage et cosmopolite, il s'observe notamment sur la pomme de terre, la betterave, le chou, des plantes de serre (laitue, chicorée, poivron, aubergine, concombre), des plantes sauvages et des cultures florales: cinéraire, chrysanthème, dahlia, oeillet, etc.. Le clone de puceron, fourni par le laboratoire de biologie des plantes et contrôle des insectes ravageurs de l'Université de Picardie Jules Verne à Amiens, est élevé en chambre climatisée sur pomme de terre.

Le protocole mis en oeuvre et le milieu nutritif sont identiques à ceux décrits précédemment pour *Myzus persicae à* l'exception du stade larvaire utilisé. Compte tenu de la taille de ces deux espèces, par souci de laisser un espace suffisant pour les larves dans le dispositif expérimental, des larves de premier stade (L1) sont utilisées pour ce type de test.

En ce qui concerne les tests de toxicité, aucune modification n'a été apportée au protocole pour les tests sur *Macrosiphum euphorbiae.* En revanche, compte ténu de la taille et de la sensibilité au stress *d'Acyrthosiphon pisum,* le nombre d'individus par boîte a été ramené à 10, le nombre de répétitions par dose testée relevé à 4, et aucun changement de milieu n'est effectué en cours de test, ce qui aurait eu pour conséquence de provoquer une perturbation trop importante des pucerons.

**6.2. Résultats**

**6.2.1. Effet répulsif et toxicité du 3,5-diCQ sur *Acyrtosiphon pisum***

6.2.1.1 Effet phagorépulsif

Les résultats obtenus (Figure 21) mettent en évidence un effet répulsif significatif du 3, 5-diCQ dès la dose 0, 03125 mM.

6.2.1.2. Effet toxique au cours de développement larvaire

La figure 22 met en évidence un effet très marqué du 3, 5-diCQ sur le développement larvaire *d'A. pisum* puisque la mortalité dépasse 50% dès 0, 03125 mM en 2 jours et atteint 100% dès 0, 25 mM en 4 jours. En revanche, elle n'excède jamais les 20% sur Ap3. Ce taux plus important de mortalité sur milieu témoin par rapport à celui observé pour *Myzus persicae* s'explique par les pertes engendrées par la manipulation de ce puceron très fragile.

Ces résultats suggèrent que la dose minimale pour détecter des effets répulsif et toxique sur cette espèce de puceron pourrait être inférieure à 0, 03125 mM, dose pour laquelle aucun effet toxique ou répulsif n'est observé chez *M. persicae.*

**6.2.2 Effet répulsif et toxicité du 3,5-diCQ sur *Macrosiphum euphorbiae***

6.2.2.1. Effet phagorépulsif

Les résultats obtenus (Figure 23) mettent en évidence un effet répulsif hautement significatif du 3, 5-diCQ sur *M. euphorbiae* dès la dose 0, 125 mM.

6.2.2.2. Effet toxique au cours du développement larvaire

La figure 24 met en évidence un effet très marqué du 3, 5-diCQ sur le développement larvaire de *Macrosiphum euphorbiae* puisque la mortalité atteint 100% en 3 à 7 jours pour les doses 0, 5 et 1 mM, et quasiment 100% en 6 jours pour la dose 0;25 mM.

## Revendications

1. Procédé de préparation de composés de formule (I)

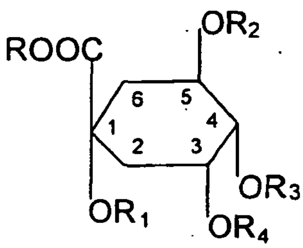

dans laquelle

- R représente un atome d'hydrogène ou un groupement méthyle
- $R_1$, $R_2$ et $R_4$ représentent chacun indépendamment l'un de l'autre un atome d'hydrogène ou un groupe caféoyle,
- $R_3$ représente un hydrogène, un groupe caféoyle ou un groupe succinyle,

à condition que deux au moins parmi $R_1$ à $R_4$ représentent un groupe caféoyle et que $R_3$ représente un groupement succinyle seulement si $R_2$ et $R_4$ représentent un groupe caféoyle
à partir de racines non tubérisées comprenant les étapes suivantes :

a) prélèvement des racines non tubérisées ou récupération de l'exsudat racinaire,

b) extraction par un ou plusieurs solvants organiques des composés phénoliques,
c) récupération de l'extrait brut,
d) purification éventuelle dudit extrait obtenu à l'étape c),
e) optionnellement, isomérisation spontanée dudit extrait en condition de pH alcalin,

lesdits composés de formule (I) étant sous la forme de régio ou stéréoisomères ou de leurs mélanges, les racines non tubérisées étant issues de plantes du genre *Ipomoea* (convolvulacées) ou des genres de la même famille *Argyreia, Calycobolus, Calystegia, Convolvulus, Dichondra, Erycibe, Evolvulus, Iseia, Jacquemontia, Maripa, Merremia, Mina, Operculina, Porana, Stictocardia,* ou *Turbina.*

**2.** Procédé selon la revendication 1 **caractérisé en ce que** les racines non tubérisées sont issues de plantes choisies dans le groupe comprenant la patate douce (*Ipomoea batatas*), le volubilis (*Ipomoea purpurea*), le liseron d'eau (*Ipomoea aquatica*), le volubilis des jardins (*Ipomoea indica*), l'Ipomée Scarlett O'Hara (*Ipomoea nil*) et le liseron des haies (*Calystegia sepium).*

**3.** Procédé selon l'une quelconque des revendications 1 ou 2, **caractérisé en ce que** les racines non tubérisées sont issues *d'Ipomoea batatas.*

**4.** Procédé selon l'une quelconque des revendications précédentes, **caractérisé en ce que** les racines non tubérisées sont produites par marcottage, bouturage ou par semis.

**5.** Procédé selon l'une quelconque des revendications précédentes, **caractérisé en ce que** des racines non tubérisées sont produites par culture en milieu liquide, à la lumière et en situation de carence minérale.

**6.** Procédé selon l'une quelconque des revendications précédentes, **caractérisé en ce que** le composé de formule (I) est choisi parmi les différentes formes isomères des acides dicaféoylquiniques, les analogues triacylés, ses analogues méthylés, l'acide 4-succinyl-3, 5-dicaféoylquinique ou leurs mélanges.

**7.** Procédé selon la revendication 6, **caractérisé en ce que** les isomères des acides dicaféoylquiniques sont choisis parmi l'acide 3, 5-dicaféoylquinique (3, 5-diCQ), la cynarine (1, 3-diCQ), l'acide 1, 5-dicaféoylquinique (1, 5-diCQ), l'acide 3, 4-dicaféoylquinique (3, 4-diCQ) et l'acide 4, 5-dicaféoylquinique (4, 5-diCQ).

**8.** Procédé selon la revendication 6, **caractérisé en ce que** l'analogues triacylés des acides dicaféoylquiniques est l'acide 3, 4, 5-tricaféoylquinique (3, 4, 5-triCQ).

**9.** Procédé selon la revendication 6, **caractérisé en ce que** les analogues méthylés des acides dicaféoylquiniques sont choisis parmi le méthyl 3, 5-dicaféoylquinate, le méthyl 3, 4-dicaféoylquinate, le méthyl 4, 5-dicaféoylquinate.

**10.** Procédé de préparation d'un composé de formule (I) selon l'une quelconque des revendications précédentes comprenant en outre les étapes suivantes :

a) prélèvement des racines non tubérisées issues de tubercules, boutures, semis ou marcottes,
b) congélation dans l'azote liquide des racines prélevées à l'étape a),
c) lyophilisation des racines congelées à l'étape b),
d) broyage dans l'azote liquide des racines lyophilisées puis lyophilisation pour obtenir une poudre sèche,
e) extraction des composés phénoliques par un solvant organique en 1 à 4 fois, par agitation à froid,
f) rinçage du dernier résidu de l'étape e) par le même solvant organique que celui utilisé à l'étape e) puis évaporation du solvant présent dans l'extrait jusqu'à obtention d'une phase aqueuse,
g) optionnellement, extraction liquide/liquide volume à volume par un solvant apolaire, par plusieurs extractions successives après addition d'un sel et d'un acide dans la phase aqueuse,
h) concentration à sec de la phase aqueuse obtenue à l'étape f) ou de la phase organique obtenue à l'étape optionnelle g) après adjonction d'un agent de séchage pour éliminer l'eau résiduelle et filtration, puis reprise du résidu sec par un solvant organique,
i) séparation par CLHP semi-préparative en phase inverse et collecte de la fraction contenant le 3,5-diCQ,
j) concentration à l'évaporateur rotatif de la fraction obtenue à l'étape i) jusqu'à l'obtention d'une phase aqueuse,
k) extraction liquide/liquide par un solvant organique (volume à volume) par plusieurs extractions successives après addition d'un sel, dans la phase aqueuse pour faciliter l'extraction dans le solvant apolaire de 3,5-diCQ,
l) concentration à sec de la phase organique, après adjonction d'un agent de séchage, filtration pour éliminer

l'eau résiduelle, et reprise par un solvant organique,
m) précipitation au froid de la molécule par ajout d'eau (au moins 3 volumes pour 1 volume de solvant organique utilisé à l'étape précédente), congélation de l'extrait dans l'azote liquide et lyophilisation pour obtenir la molécule sous forme de poudre sèche.

**11.** Procédé de préparation d'un composé de formule (I)selon la revendication 10 **caractérisé en ce que** l'étape g) est réalisée par extraction, soit par l'acétate d'éthyle soit par le diéthyléther (volume à volume) après addition de NaCl ou de sulfate d'ammonium et d'acide métaphosphorique dans la phase aqueuse et l'étape k) est réalisée par extraction, soit par l'acétate d'éthyle soit par le diéthyléther (volume à volume) après addition de NaCl ou de sulfate d'ammonium dans la phase aqueuse.

**Patentansprüche**

**1.** Verfahren zur Herstellung von Verbindungen der Formel (I)

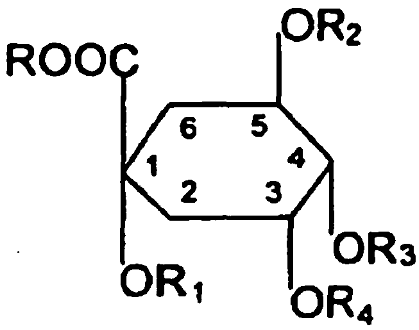

Worin

- $R_1$ für ein Wasserstoffatom oder eine Methylgruppe steht,
- $R_1$, $R_2$ und $R_4$ unabhängig voneinander jeweils für ein Wasserstoffatom oder eine Caffeoylgruppe stehen,
- $R_3$ für einen Wasserstoff, eine Caffeoylgruppe oder eine Succinylgruppe stehen, unter der Voraussetzung, dass mindestens zwei aus $R_1$ bis $R_4$ für eine Caffeoylgruppe stehen und dass $R_3$ nur dann für eine Succinylgruppe steht, wenn $R_2$ und $R_4$ für eine Caffeoylgruppe stehen,

aus nicht knollenförmigen Wurzeln, umfassend die folgenden Schritte:

a) Entnehmen der nicht knollenförmigen Wurzeln oder Gewinnen des Wurzelexsudats,
b) Extrahieren der Phenolverbindungen mit einem oder mehreren organischen Lösemitteln,
c) Gewinnen des Rohextrakts,
d) gegebenenfalls Reinigen des in Schritt c) erhaltenen Extrakts,
e) optional spontanes Isomerisieren des Extrakts unter alkalischen pH-Bedingungen,

wobei die Verbindungen der Formel (I) die Form von Regio- oder Stereoisomeren oder deren Gemischen aufweisen, wobei die nicht knollenförmigen Wurzeln aus Pflanzen der Gattung *Ipomoea* (Convolvulaceae) oder der Gattungen aus der gleichen Familie *Argyreia, Calycobolus, Calystegia, Convolvulus, Dichondra, Erycibe, Evolvulus, Iseia, Jacquemmontia, Maripa, Merremia, Mina, Operculina, Porana, Stictocardia* oder *Turbina* stammen.

**2.** Verfahren nach Anspruch 1 oder 2, **dadurch gekennzeichnet, dass** die nicht knollenförmigen Wurzeln aus Pflanzen stammen, ausgewählt aus der Gruppe umfassend Süßkartoffel (*Ipomoea batatas*), Volubilis (*Ipomoea purpurea*), Wasserspinat *(Ipomoea a quatica),* Kaiserwinde (*Ipomoea indica*), Blaue Prunkwinde Scarlett O'Hara (*Ipomoea nil)* und Echte Zaunwinde *(Calystegia sepium).*

**3.** Verfahren nach einem der Ansprüche 1 bis 2, **dadurch gekennzeichnet, dass** die nicht knollenförmigen Wurzeln von *Ipomoea batatas* stammen.

**4.** Verfahren nach einem der vorhergehenden Ansprüche, **dadurch gekennzeichnet, dass** die nicht knollenförmigen Wurzeln durch Vermehren durch Absenken, Vermehren durch Stecklinge oder durch Aussäen hergestellt werden.

5. Verfahren nach einem der vorhergehenden Ansprüche, **dadurch gekennzeichnet, dass** die nicht knollenförmigen Wurzeln durch Kultur in flüssigem Medium unter Licht und unter Mineralmangelbedingungen hergestellt werden.

6. Verfahren nach einem der vorhergehenden Ansprüche, **dadurch gekennzeichnet, dass** die Verbindung der Formel (I) ausgewählt ist aus den verschiedenen isomeren Formen der Dicaffoylchinasäuren, den triacylierten Analoga, ihren methylierten Analoga, 4-Succinyl-3, 5-dicaffeoylchinasäure oder deren Gemischen.

7. Verfahren nach Anspruch 6, **dadurch gekennzeichnet, dass** die Isomere der Dicaffeoylchinasäuren ausgewählt sind aus 3, 5-Dicaffeoylchinasäure (3, 5-diCQ), Cynarin (1, 3-diCQ), 1, 5-Dicaffeoylchinasäure (1, 5-diCQ), 3, 4-Dicaffeoylchinasäure (3, 4-diCQ) und 4, 5-Dicaffeoylchinasäure (4, 5-diCQ).

8. Verfahren nach Anspruch 6, **dadurch gekennzeichnet, dass** das triacylierte Analogon der Dicaffeoylchinasäure 3, 4, 5-Tricaffeoylchinasäure (3, 4, 5-triCQ) ist.

9. Verfahren nach Anspruch 6, **dadurch gekennzeichnet, dass** die methylierten Analoga der Dicaffeoylchinasäuren ausgewählt sind aus Methyl-3, 5-dicaffeoylchinat, Methyl-3, 4-dicaffeoylchinat, Methyl-4, 5-dicaffeoylchinat.

10. Verfahren zur Herstellung einer Verbindung der Formel (I) nach einem der vorhergehenden Ansprüche, das ferner die folgenden Schritte umfasst:

a) Entnehmen von nicht knollenförmigen Wurzeln, die aus Knollen, Stecklingen, Samen oder Ablegern stammen,
b) Einfrieren der in Schritt a) entnommenen Wurzeln in Flüssigstickstoff,
c) Lyophilisieren der in Schritt b) eingefrorenen Wurzeln,
d) Zermahlen der lyophilisierten Wurzeln in Flüssigstickstoff, dann Lyophilisieren, um ein trockenes Pulver zu erhalten,
e) Extrahieren der Phenolverbindungen mit einem organischen Lösemittel in 1 bis 4 Durchgängen mittels Kaltrühren,
f) Spülen des letzten Rückstands aus Schritt e) mit dem gleichen organischen Lösemittel wie dem, das in Schritt e) verwendet wurde, dann Verdampfen des in dem Extrakt vorhandenen Lösemittels bis eine wässrige Phase erhalten wird,
g) optional Flüssig-flüssig-Extrahieren Volumen für Volumen mit einem apolaren Lösemittel, durch mehrere aufeinander folgende Extraktionen nach Zugeben eines Salzes und einer Säure in die wässrige Phase,
h) Trockenkonzentrieren der in Schritt f) erhaltenen wässrigen Phase oder der im optionalen Schritt g) erhaltenen organischen Phase nach Hinzufügen eines Trocknungsmittels zum Entfernen des Restwassers und Filtrieren, dann Aufnehmen des trockenen Rückstands mit einem organischen Lösemittel,
i) Trennen mittels semi-präparativer Umkehrphasen-HPCL und Sammeln der 3,5-diCQ enthaltenden Fraktion,
j) Konzentrieren der in Schritt d) erhaltenen Fraktion mit einem Rotationsverdampfer bis eine wässrige Phase erhalten wird,
k) Flüssig-flüssig-Extrahieren mit einem organischen Lösemittel (Volumen für Volumen) durch mehreren aufeinander folgende Extraktionen nach Zugeben eines Salzes in die wässrige Phase, um die Extraktion von 3,5-diCQ in dem apolaren Lösemittel zu erleichtern,
l) Trockenkonzentrieren der organischen Phase nach Hinzufügen eines Trocknungsmittels, Filtrieren zum Entfernen des Restwassers und Aufnehmen mit einem organischen Lösemittel,
m) Kaltfällen des Moleküls durch Zufügen von Wasser (mindestens 3 Volumen für 1 Volumen des im vorhergehenden Schritt verwendeten organischen Lösemittels), Einfrieren des Extrakts in Flüssigstickstoff und Lyophilisieren, um das Molekül in Form eines trockenen Pulvers zu erhalten.

11. Verfahren zur Herstellung einer Verbindung der Formel (I) nach Anspruch 10, **dadurch gekennzeichnet, dass** der Schritt g) entweder durch Extrahieren mit Ethylacetat oder mit Diethylether (Volumen für Volumen) nach Zugeben von NaCl oder Ammoniumsulfat und Metaphosphorsäure in die wässrige Phase ausgeführt wird und Schritt k) entweder durch Extrahieren mit Ethylacetat oder mit Diethylether (Volumen für Volumen) nach der Zugabe von NaCl oder von Ammoniumsulfat in die wässrige Phase ausgeführt wird.

## Claims

1. A method for preparing compounds of Formula (I)

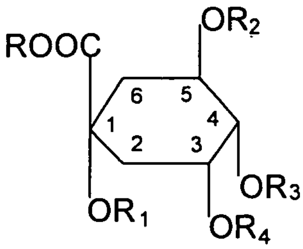

wherein
R represents a hydrogen atom or a methyl group,
R1, R2, and R4 each represent, independently of each other, a hydrogen atom or a caffeoyl group,
R3 represents a hydrogen, a caffeoyl group, or a succinyl group,
provided that at least two of R1 through R4 represent a caffeoyl group, and that R3 represents a succinyl group only if R2 and R4 represent a caffeoyl group,
from nontuberized roots comprising the following steps:

a) taking of nontuberized roots or recovery of root exudate thereof,
b) extraction of phenolic compounds by one or several organic solvents, and
c) recovery of the raw extract.
d) eventual purification of said extract obtained in Step c).
e) optionally, spontaneous isomerization of said extracts under alkaline pH conditions,

said Formula (I) compounds being in the form of regio- or stereoisomers, or mixtures thereof, said nontuberized roots originate from plants from the genera *Ipomoea* () or the genera of the same family *Argyreia, Calycobolus, Calystegia, Convolvulus, Dichondra, Erycibe, Evolvulus, Ipomoea, Iseia, Jacquemontia, Maripa, Merremia, Mina, Operculina, Porana, Stictocardia, or Turbina.*

2. The method according to claim 1, **characterized in that** said nontuberized roots originate from plants selected from the group comprising the sweet potato (Ipomoea batatas), morning glory (*Ipomoea purpurea*), water spinach (*Ipomoea aquatica*), oceanblue morning glory (*Ipomoea indica*), Scarlett O'Hara morning glory (*Ipomoea nil*), and hedge bindweed *(Calystegi sepium).*

3. The method according to one of claims 1 or 2, **characterized in that** said nontuberized roots originate from *Ipomoea batatas.*

4. The method according to any one of preceding claims, **characterized in that** nontuberized roots are produced by layering, cutting or by seeding.

5. The method according to any one of preceding claims, **characterized in that** nontuberized roots are produced by growing in a liquid medium, under light, and under mineral deficiency conditions.

6. The method according to any one of preceding claims, **characterized in that** the Formula (I) compound is selected from different isomeric forms of dicaffeoylquinic acids, the triacylated analogues, its methylated analogues, 4-succinyl-3, 5-dicaffeoylquinic acid, or a mixture thereof.

7. The method according to claim 6, **characterized in that** the isomers of dicaffeoylquinic acids are chosen from 3, 5-dicaffeoylquinic (3, 5-diCQ) acid, cynarin (1, 3-diCQ), 1, 5-dicaffeoylquinic (1, 5-diCQ) acid, 3, 4-dicaffeoylquinic (3, 4-diCQ) acid, 4, 5-dicaffeoylquinic (4, 5-diCQ) acid.

8. The method according to claim 6, **characterized in that** the triacylated analogues of dicaffeoylquinic acids is 3, 4, 5-tricaffeoylquinic (3, 4, 5-triCQ) acid.

9. The method according to claim 6, **characterized in that** the methylated analogues of dicaffeoylquinic acids are chosen from methyl 3, 5-dicaffeoylquinate, methyl 3, 4-dicaffeoylquinate, methyl 4, 5-dicaffeoylquinate.

10. The method for preparing a formula (I) compound according to any one of preceding comprising claims, comprising

furthermore the following steps:

a) taking the nontuberized roots originating from tubers, cuttings, seedlings, or layers,
b) freezing said roots sampled in Step a) in liquid nitrogen,
c) freeze-drying said roots frozen in Step b),
d) grinding said freeze-dried roots in liquid nitrogen, then lyophilizing them in order to obtain a dry powder,
e) extracting phenolic compounds by an organic solvent in 1 to 4 passes, via cold stirring,
f) rinsing the final residue from Step e) with the same organic solvent as the one used in Step e), then evaporating the solvent present in the extract until obtaining an aqueous phase,
g) optionally, liquid/liquid volume-to-volume extracting by an apolar solvent, via several successive extractions, after adding a salt and an acid to said aqueous phase,
h) dry concentration of the aqueous phase obtained in Step f) or of the organic phase obtained in Step g), after adding a drying agent in order to eliminate residual water and filtration, then recovering the dry residue by an organic solvent,
i) separating by semipreparative reversed-phase HPLC and collecting the fraction containing 3,5-diCQ,
j) concentrating the fraction obtained in Step i) in rotary evaporator until an aqueous phase is obtained,
k) liquid/liquid extracting (volume-to volume) by an organic solvent via several successive extractions, after adding a salt to said aqueous phase, in order to facilitate 3,5-diCQ extraction into the apolar solvent,
l) dry concentration of the organic phase, after adding a drying agent and filtration in order to eliminate residual water, and recovering the dry residue using an organic solvent,
m) cold precipitating the molecule by adding water (at least 3 volumes for 1 volume of the organic solvent used in the previous step), and freezing the exudate in liquid nitrogen in order to obtain a molecule in the form of a dry powder.

**11.** The method for preparing a compound of formule (I) according to claim 10, **characterized in that** Step g) is carried out by extracting with ethyl acetate or diethylether (volume-to-volume) after adding NaCl or ammonium sulfate and metaphosphoric acid into the aqueous phase and Step k) is performed via extraction, either by ethyl acetate or by diethylether (volume to volume) after NaCl or ammonium sulfate is added into the aqueous phase.

FIGURE 1

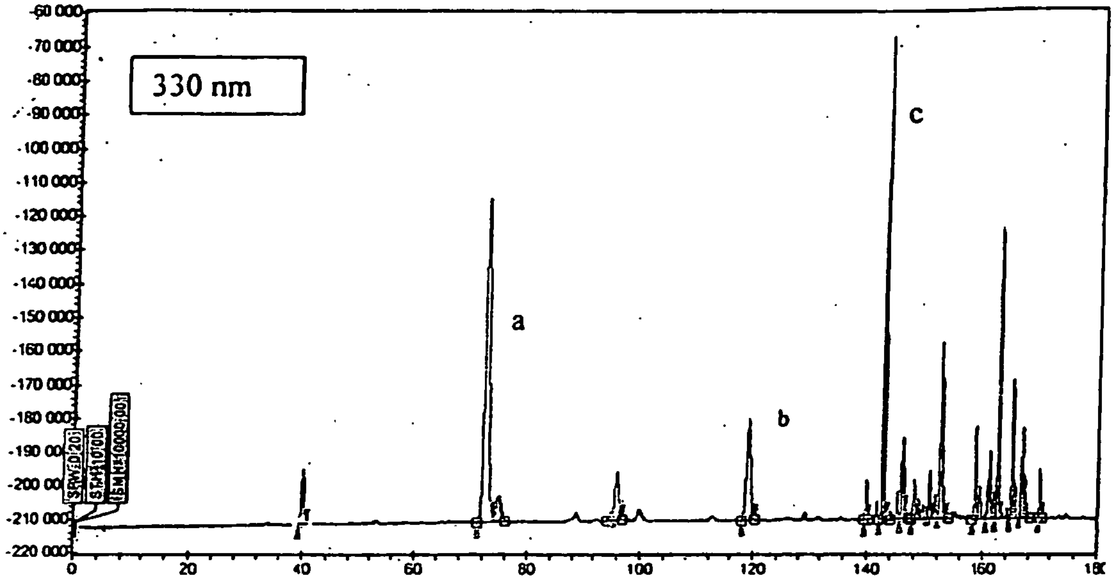
330 nm
a
b
c
-60 000
-70 000
-80 000
-90 000
-100 000
-110 000
-120 000
-130 000
-140 000
-150 000
-160 000
-170 000
-180 000
-190 000
-200 000
-210 000
-220 000
0
20
40
60
80
100
120
140
160
180

**FIGURE 2**

Figure 2A

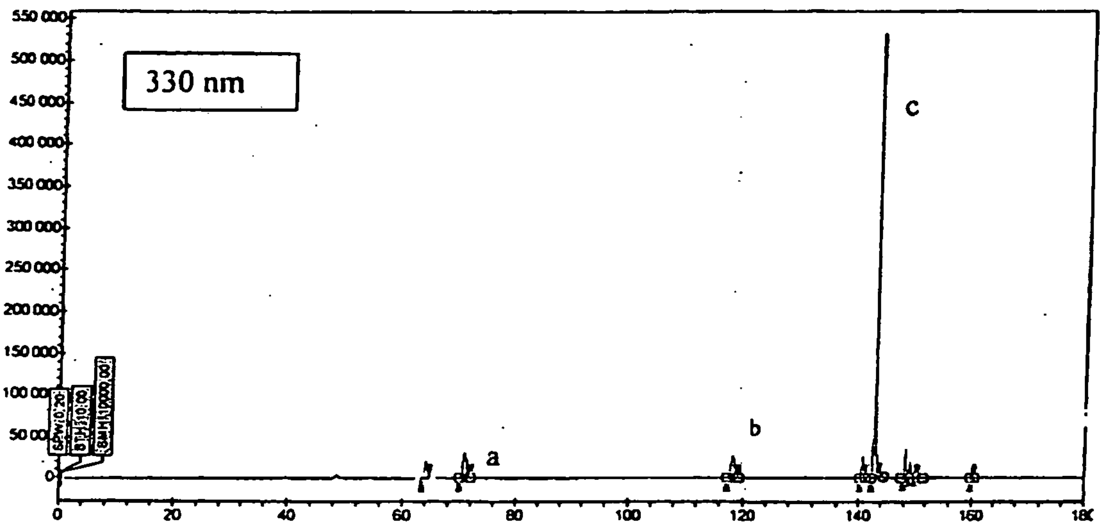

2B

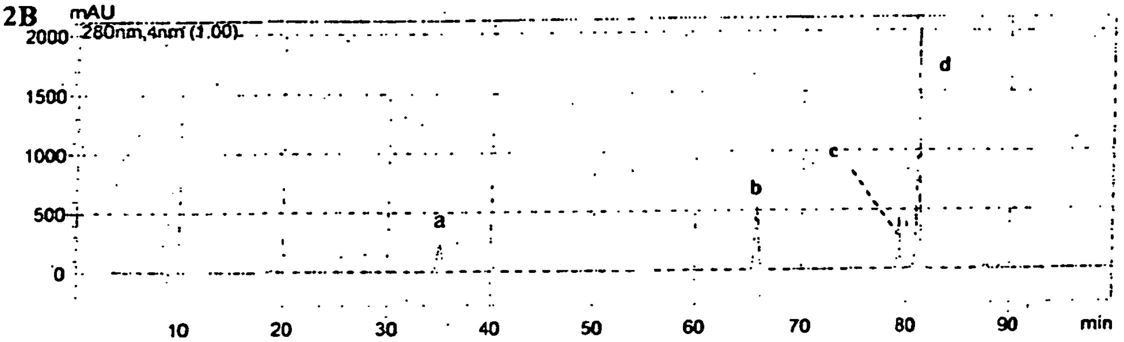
mAU
280nm,4nm (1.00)
2000
1500
1000
500
0
10
20
30
40
50
60
70
80
90
min
a
b
c
d

2C

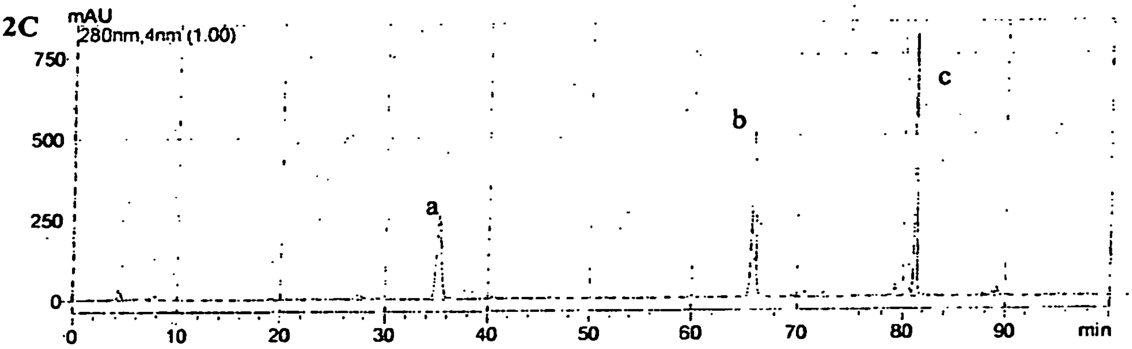
mAU
280nm,4nm (1.00)
750
500
250
0
0
10
20
30
40
50
60
70
80
90
min
a
b
c

2D

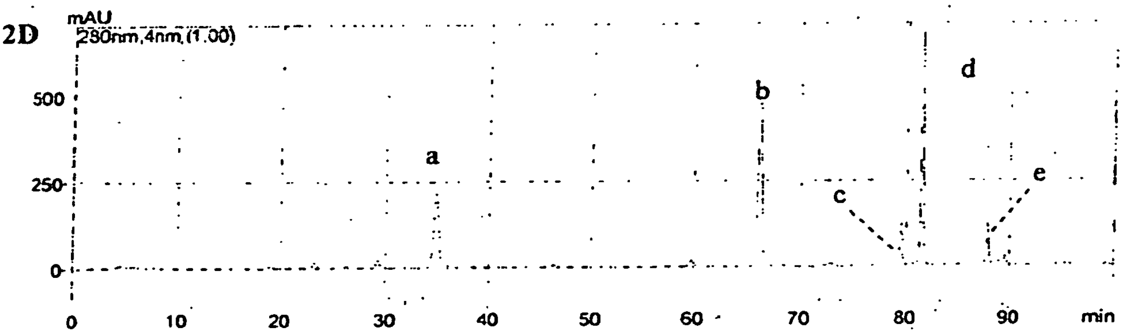
mAU
280nm,4nm (1.00)
500
250
0
0
10
20
30
40
50
60
70
80
90
min
a
b
c
d
e

2E

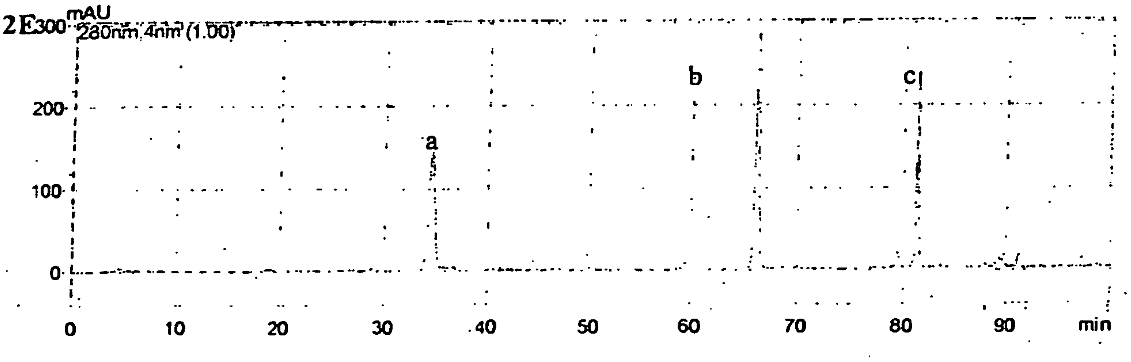
mAU
280nm,4nm (1.00)
300
200
100
0
0
10
20
30
40
50
60
70
80
90
min
a
b
c

FIGURE 3

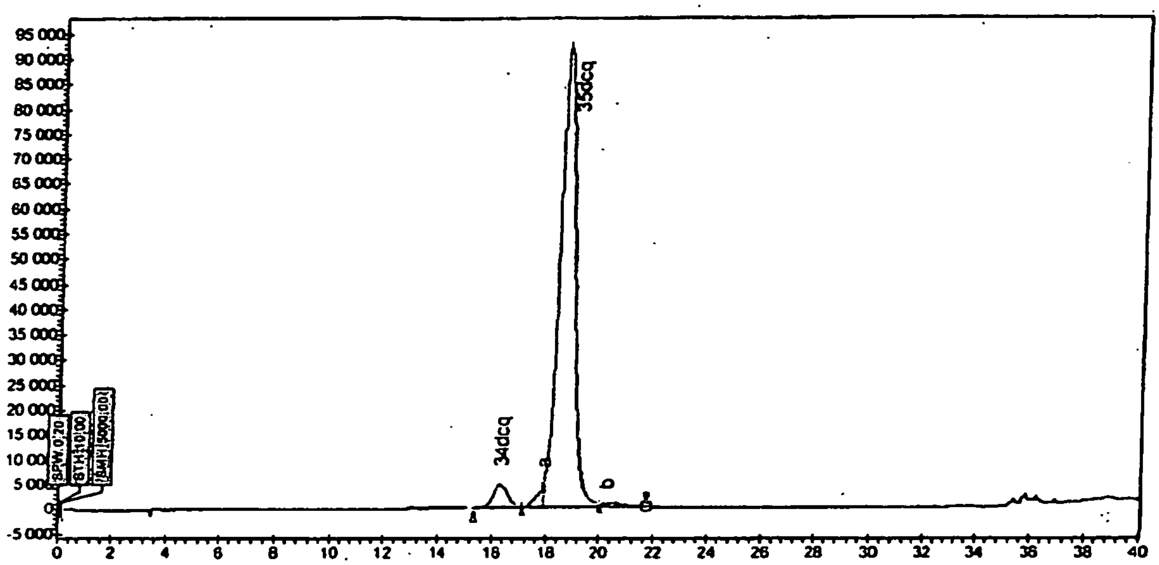
34dcq
35dcq
95 000
90 000
85 000
80 000
75 000
70 000
65 000
60 000
55 000
50 000
45 000
40 000
35 000
30 000
25 000
20 000
15 000
10 000
5 000
0
-5 000
0
2
4
6
8
10
12
14
16
18
20
22
24
26
28
30
32
34
36
38
40

**Figure 4**

| Condition | | | Matière sèche racinaire (g) | Teneur en 3,5-diCQ ($mg.g^{-1}$ MS) | Quantité de 3,5-diCQ par plante (mg) |
|---|---|---|---|---|---|
| | | Milieu/solution/éclairement | | | |
| A | | Liquide/eau/obs | 0,30 | 45,7 | 13,9 |
| B | | Liquide/eau/lum | 0,31 | 92,0 | 28,2 |
| C | | Liquide/sol 0.05 mM N/obs | 0,33 | 30,9 | 10,0 |
| D | | Liquide/sol 6 mM N/obs | 1,57 | 20,6 | 32,0 |
| E | | Perlite/eau/obs | 0,68 | 36,3 | 24,9 |
| F | | Perlite/sol 6 mM N/obs | 1,75 | 14,8 | 25,4 |

Figure 5

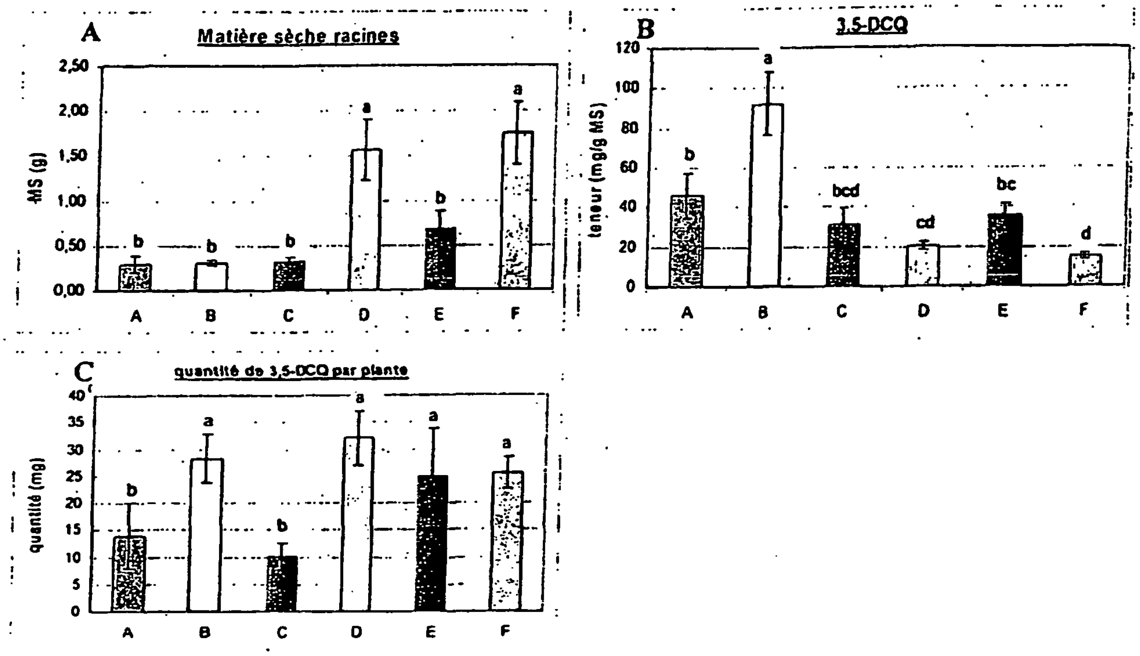
A
Matière sèche racines
MS (g)
2,50
2,00
1,50
1,00
0,50
0,00
A B C D E F
a a b b b b
B
3,5-DCQ
teneur (mg/g MS)
120
100
80
60
40
20
0
A B C D E F
a b bcd cd bc d
C
quantité de 3,5-DCQ par plante
quantité (mg)
40
35
30
25
20
15
10
5
0
A B C D E F
b a b a a a

Figure 6

| Condition | | | Matière sèche racinaire (g) | Teneur en 3,5-diCQ ($mg.g^{-1}$ MS) | Quantité de 3,5-diCQ par plante (mg) |
|---|---|---|---|---|---|
| | | Milieu/solution/éclairement | | | |
| E | | Liquide/eau/obs | 0,315 | 51,13 | 16,10 |
| F | | Liquide/sol 6 mM N, 1 mM P/obs | 0,677 | 25,11 | 17,38 |
| G | | Liquide/sol 0 mM N, 1 mM P/obs | 0,469 | 28,11 | 12,95 |
| H | | Liquide/sol 6 mM N, 0 mM P/obs | 0,445 | 35,09 | 16,85 |

Figure 7

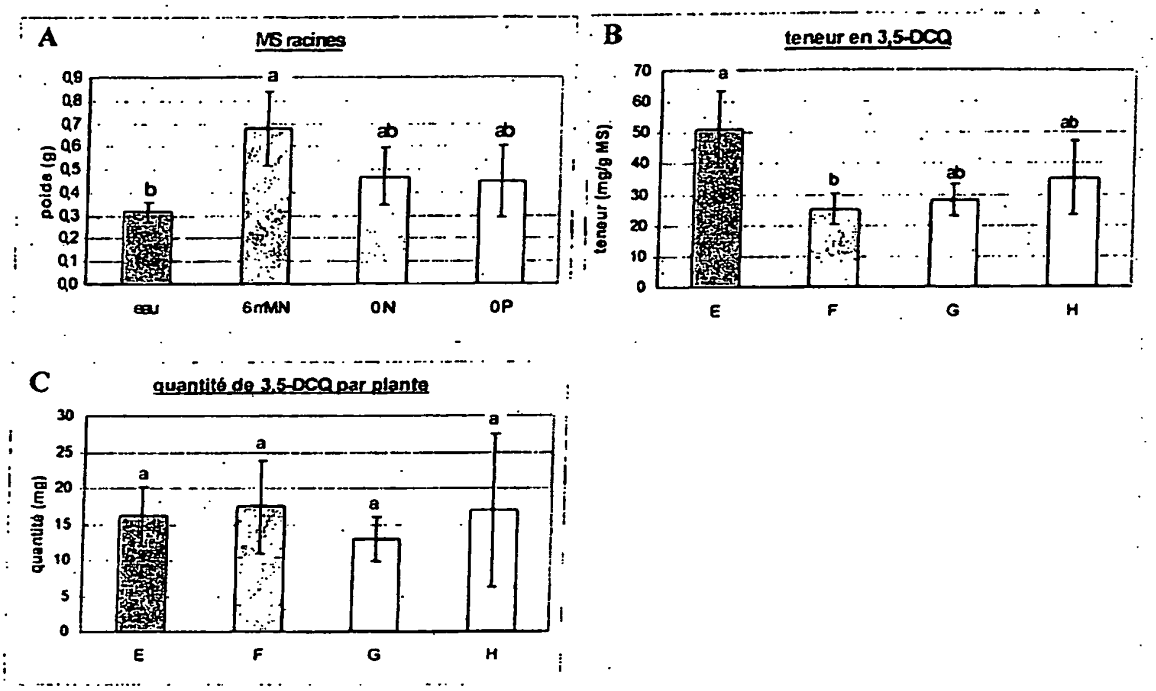
A
MS racines
poids (g)
0,0
0,1
0,2
0,3
0,4
0,5
0,6
0,7
0,8
0,9
b
a
ab
ab
eau
6mMN
0N
0P
B
teneur en 3,5-DCQ
teneur (mg/g MS)
0
10
20
30
40
50
60
70
a
b
ab
ab
E
F
G
H
C
quantité de 3,5-DCQ par plante
quantité (mg)
0
5
10
15
20
25
30
a
a
a
a
E
F
G
H

Figure 8

| Condition | | | Matière sèche (g) | | Teneur 3,5-diCQ ($mg.g^{-1}$ MS) | | Q 3,5-diCQ/plante (mg) | | |
|---|---|---|---|---|---|---|---|---|---|
| | boutures | marcottes | boutures | marcottes | boutures | marcottes | boutures | marcottes | Total par plant |
| A | Eau | Eau | 0,36 | 0,77 | 47,4 | 31,9 | 16,8 | 24,5 | 41.3 |
| B | Eau | Solution | 0,31 | 1,25 | 44,7 | 11,6 | 13,6 | 14,7 | 28.3 |
| C | Solutions | Solution | 0,99 | 1,22 | 16,2 | 10,2 | 17,4 | 13,6 | 31.0 |
| D | Solution | Eau | 2,03 | 0,74 | 15,5 | 35,5 | 31,1 | 26,2 | 57.3 |

Figure 9

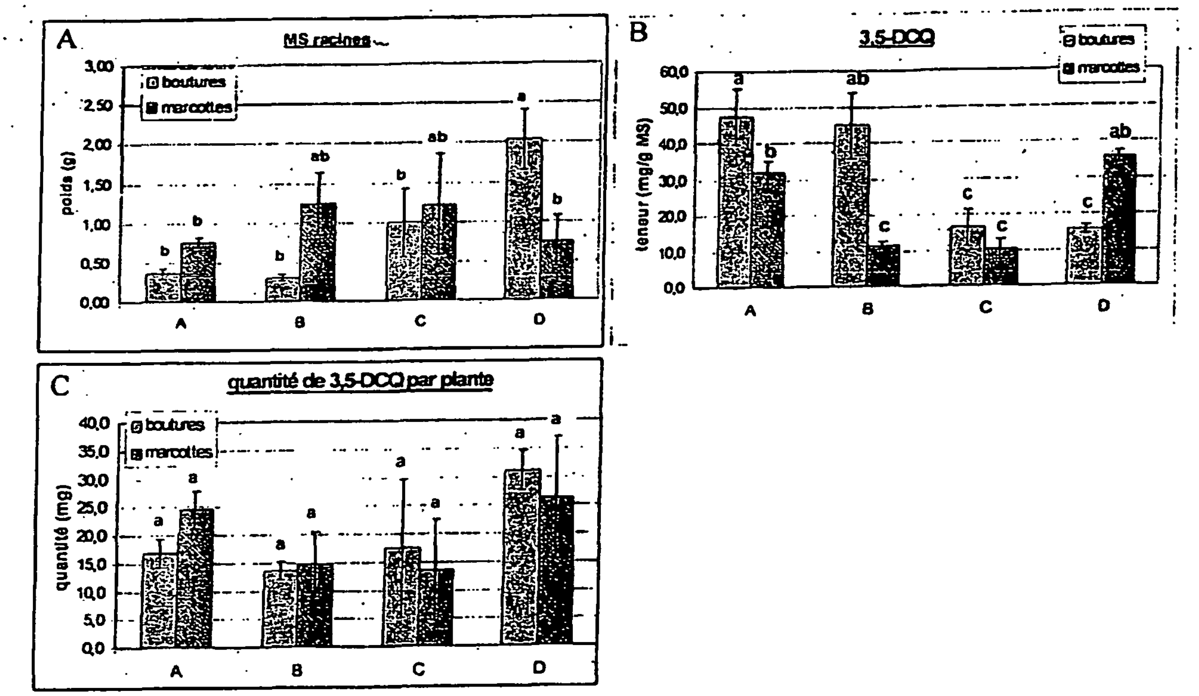
A
MS racines
boutures
marcottes
poids (g)
3,00
2,50
2,00
1,50
1,00
0,50
0,00
A
B
C
D
B
3,5-DCQ
boutures
marcottes
teneur (mg/g MS)
60,0
50,0
40,0
30,0
20,0
10,0
0,0
C
quantité de 3,5-DCQ par plante
quantité (mg)
40,0
35,0
30,0
25,0
20,0
15,0
10,0
5,0
0,0

## FIGURE 10

| | Masse Moléculaire | mM | Pour 100 mL |
|---|---|---|---|
| **Acides aminés (L)** | | | mg |
| Alanine | 89,09 | 20,06 | 178,71 |
| β-alanine | 89,1 | 0,70 | 6,22 |
| Arginine | 174,2 | 14,06 | 244,90 |
| Asparagine $H_2O$ | 150,14 | 19.88 | 298,55 |
| Acide aspartique | 133,11 | 6.63 | 88,25 |
| Cystéine | 121,16 | 2.44 | 29.59 |
| Acide glutamique | 147,13 | 10,15 | 149,36 |
| Glutamine | 146,15 | 30,49 | 445,61 |
| Glycine | 75,07 | 22,19 | 166,56 |
| Histidine HCl $H_2O$ | 209.63 | 6,49 | 136,02 |
| Isoleucine (allo free) | 131.18 | 12,56 | 164,75 |
| Leucine | 131.18 | 17,65 | 231,56 |
| Lysine HCl | 182,65 | 19,22 | 351,09 |
| Méthionine | 149,21 | 4,85 | 72,35 |
| Ornithine HCl | 168,62 | 0,56 | 9.41 |
| Phénylalanine | 165,19 | 17,83 | 294,53 |
| Proline | 115.13 | 11,23 | 129,33 |
| Sérine | 105.09 | 11,83 | 124,28 |
| Thréonine (allo free) | 119,12 | 10,67 | 127,16 |
| Tryptophane | 204,23 | 2,09 | 42,75 |
| Tyrosine | 181,19 | 2,13 | 38,63 |
| Valine | 117,15 | 16.29 | 190,85 |
| **Total** | | 260,01 | 3520,45 |
| **Saccharose** | 342,3 | 584,28 | 20000,00 |
| Rapport Saccharose/AA | | 2,25 | |
| **Vitamines** | | | (mg) |
| Acide p-aminobenzoique | | | 10,00 |
| Acide L-ascorbique | | | 100,00 |
| Biotine | | | 0,10 |
| D-Panthothénate de calcium | | | 5,00 |
| Chlorure de choline | | | 50,00 |
| Acide folique | | | 1,00 |
| i-Inositol anhydre | | | 42,00 |
| Amide nicotinique | | | 10,00 |
| Pyridoxine HCl | | | 2.50 |
| Riboflavine | | | 0.50 |
| Thiamine HCl | | | 2.50 |
| **Métaux traces** | | | (mg) |
| $CuSO_4$, $5H_2O$ | | | 0,47 |
| $FeCl_3$, $6H_2O$ | | | 4.45 |
| $MnCl_2$, $4H_2O$ | | | 0,65 |
| NaCl | | | 2,54 |
| $ZnCl_2$ | | | 0.83 |
| **Divers** | | | (mg) |
| Citrate de calcium | | | 10,00 |
| Benzoate de cholestérol | | | 2,50 |
| $MgSO_4$ $7H_2O$ | | | 242,00 |
| $KH_2PO_4$ | | | 250,00 |

**FIGURE 11**

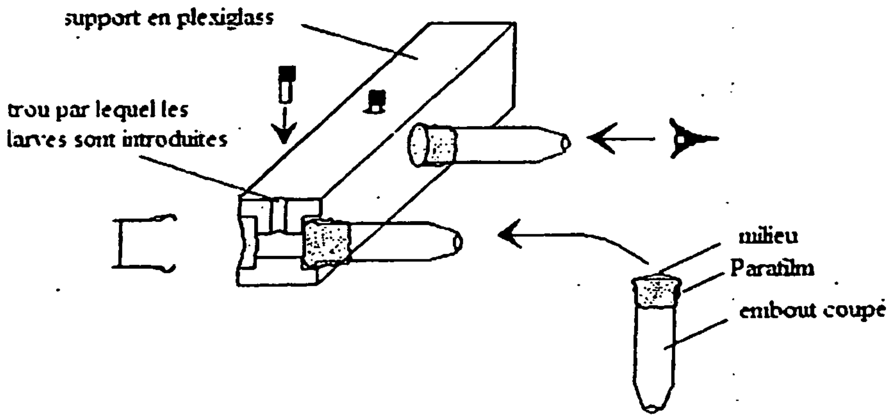
support en plexiglass
trou par lequel les larves sont introduites
milieu
Parafilm
embout coupé

# FIGURE 12

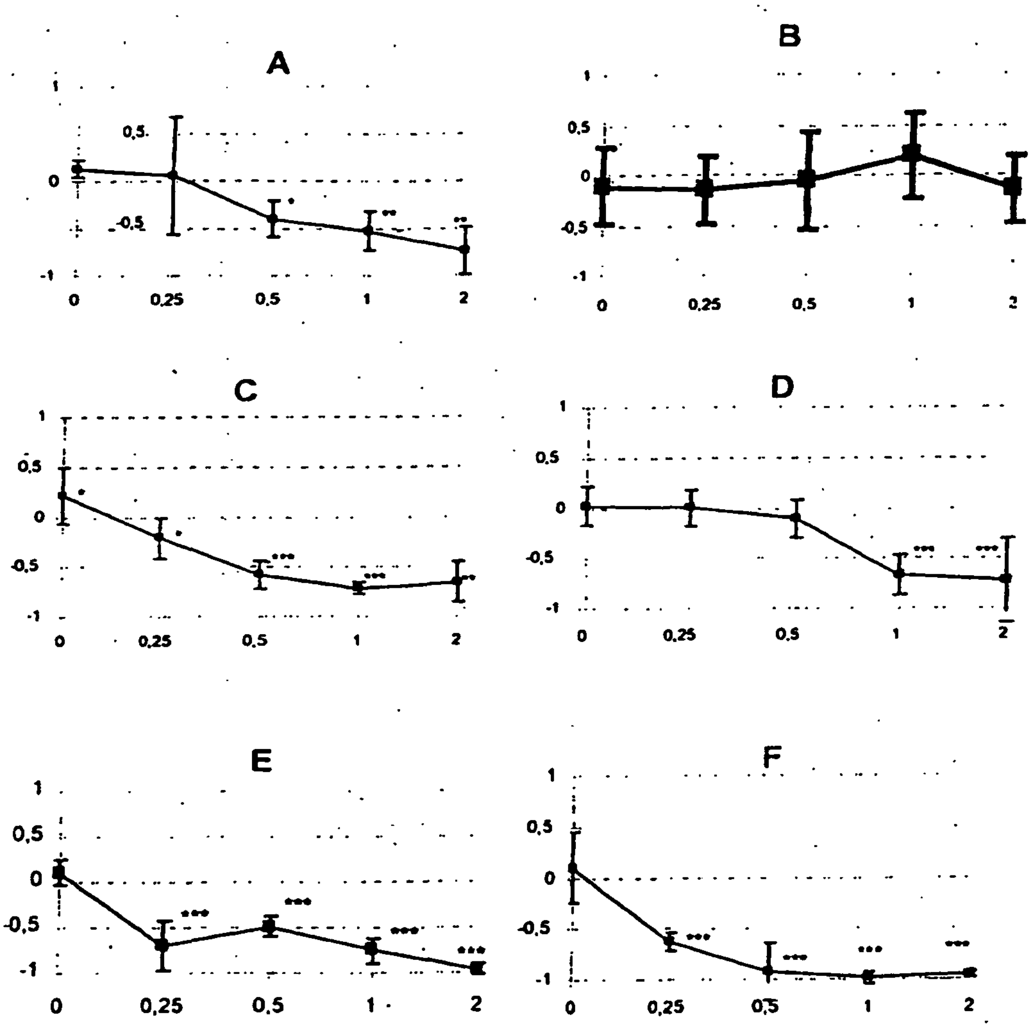

FIGURE 13

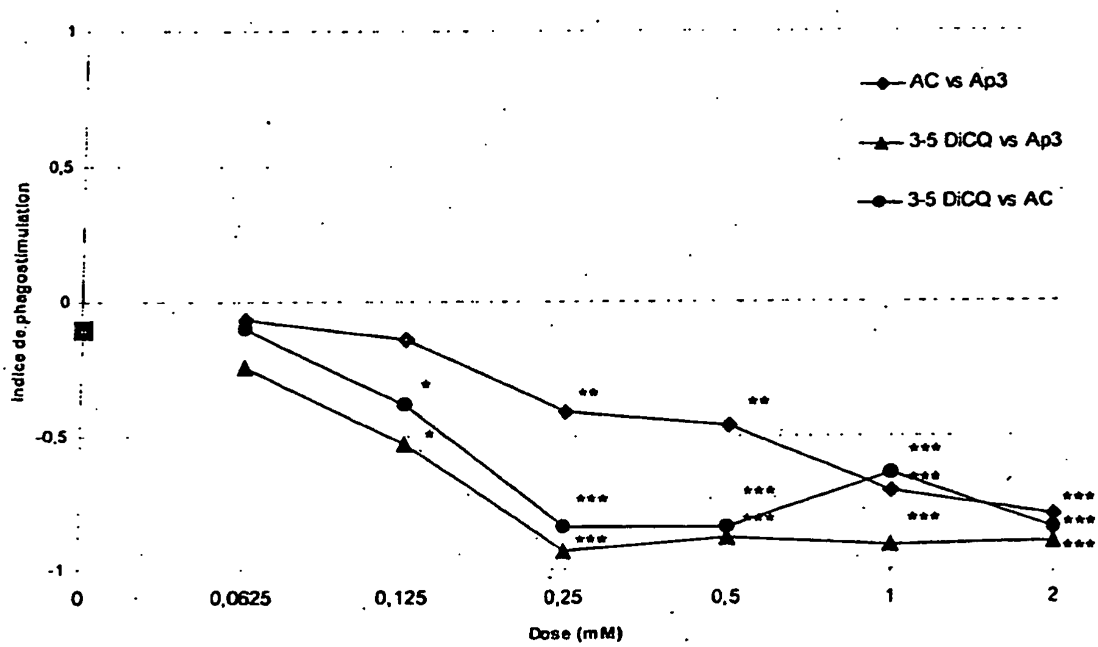
AC vs Ap3
3-5 DiCQ vs Ap3
3-5 DiCQ vs AC
Indice de phagostimulation
1
0,5
0
-0,5
-1
0
0,0625
0,125
0,25
0,5
1
2
Dose (mM)

**FIGURE 14**

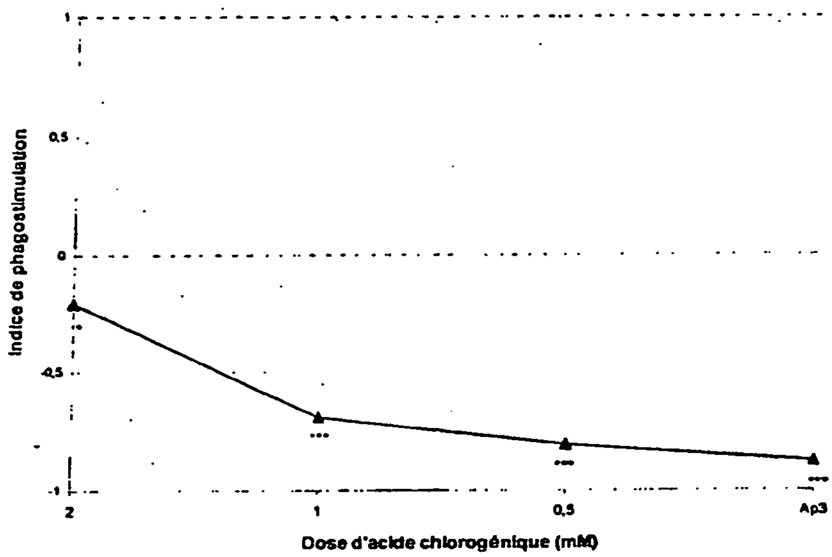

Indice de phagostimulation
1
0,5
0
-0,5
-1
2
1
0,5
Ap3
Dose d'acide chlorogénique (mM)

Figure 15

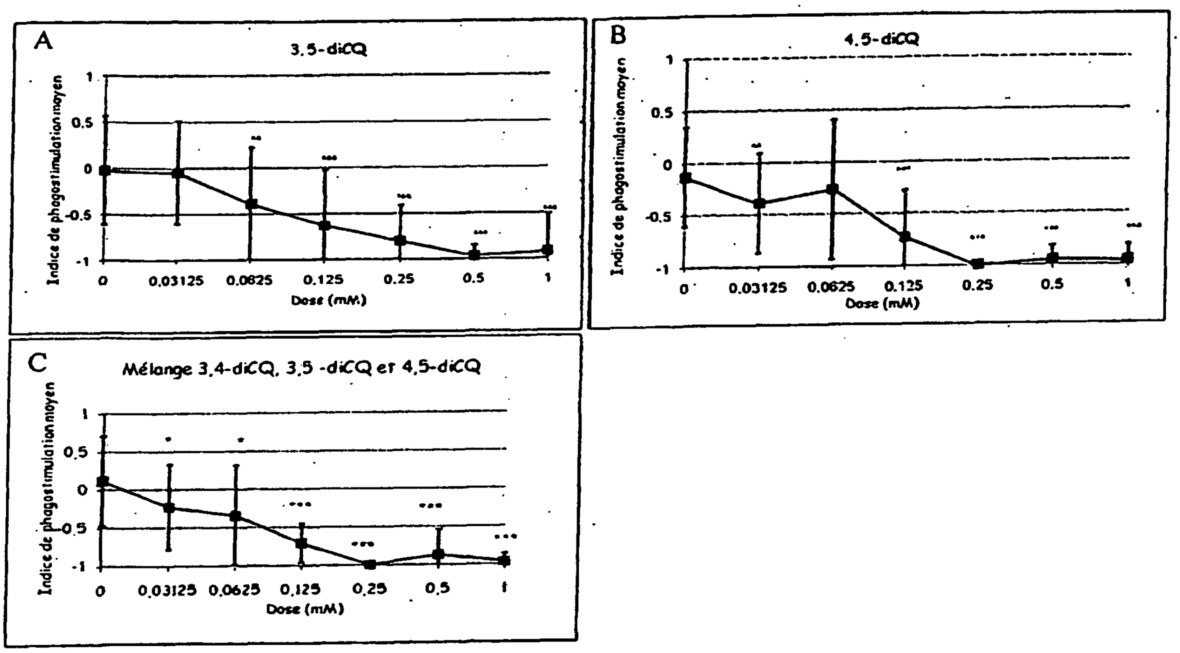
A
3,5-diCQ
Indice de phagostimulation moyen
Dose (mM)
B
4,5-diCQ
Indice de phagostimulation moyen
Dose (mM)
C
Mélange 3,4-diCQ, 3,5 -diCQ et 4,5-diCQ
Indice de phagostimulation moyen
Dose (mM)

# FIGURE 16

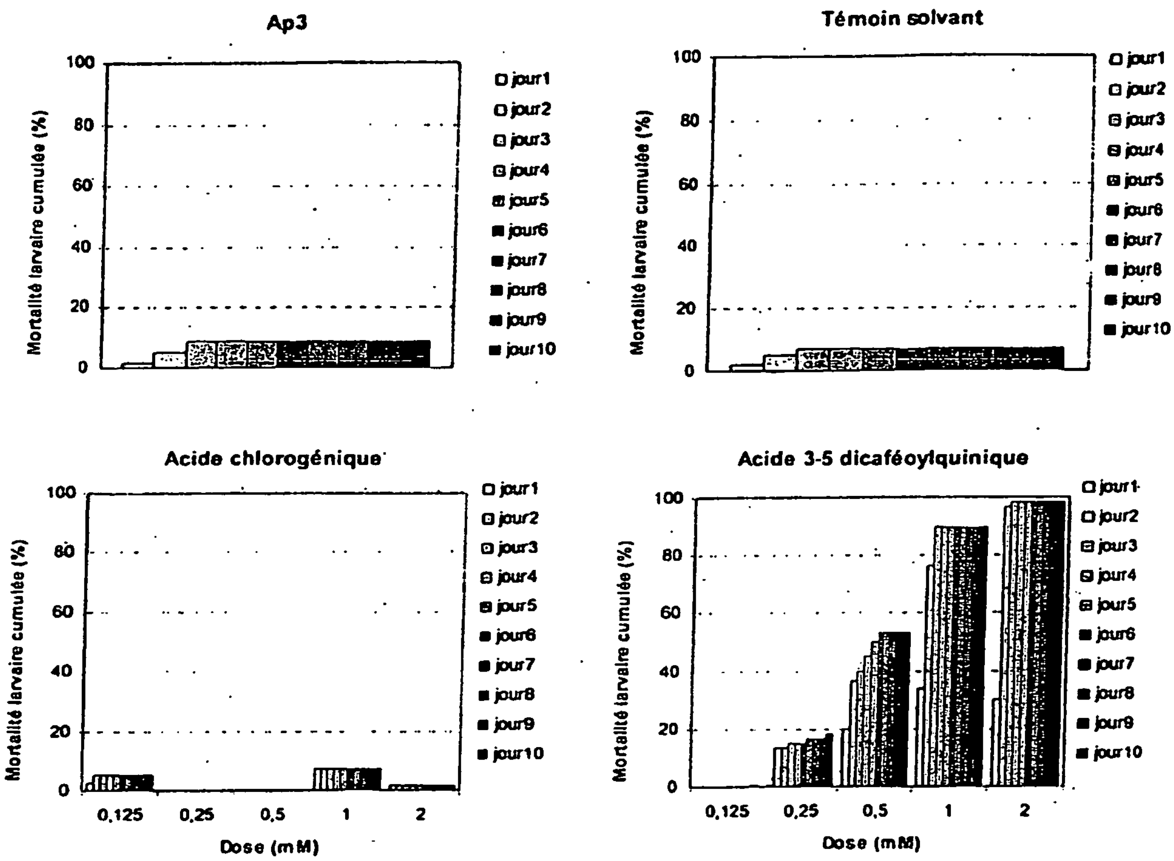
Ap3
Mortalité larvaire cumulée (%)
100
80
60
40
20
0
jour1
jour2
jour3
jour4
jour5
jour6
jour7
jour8
jour9
jour10
Témoin solvant
Mortalité larvaire cumulée (%)
100
80
60
40
20
0
jour1
jour2
jour3
jour4
jour5
jour6
jour7
jour8
jour9
jour10
Acide chlorogénique
Mortalité larvaire cumulée (%)
100
80
60
40
20
0
0,125
0,25
0,5
1
2
Dose (mM)
jour1
jour2
jour3
jour4
jour5
jour6
jour7
jour8
jour9
jour10
Acide 3-5 dicaféoylquinique
Mortalité larvaire cumulée (%)
100
80
60
40
20
0
0,125
0,25
0,5
1
2
Dose (mM)
jour1
jour2
jour3
jour4
jour5
jour6
jour7
jour8
jour9
jour10

FIGURE 17

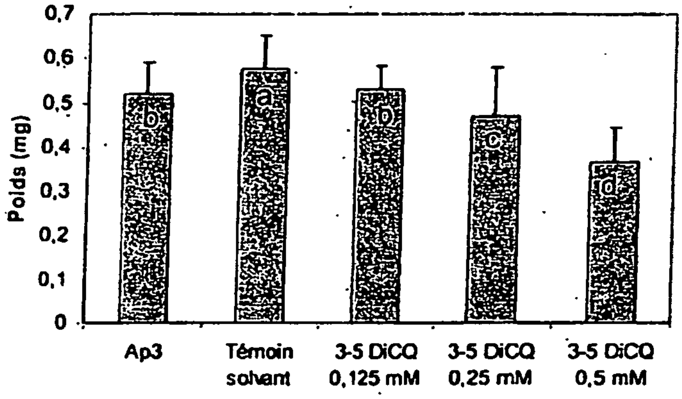
0,7
0,6
0,5
0,4
0,3
0,2
0,1
0
Poids (mg)
b
a
b
c
d
Ap3
Témoin solvant
3-5 DiCQ 0,125 mM
3-5 DiCQ 0,25 mM
3-5 DiCQ 0,5 mM

FIGURE 18

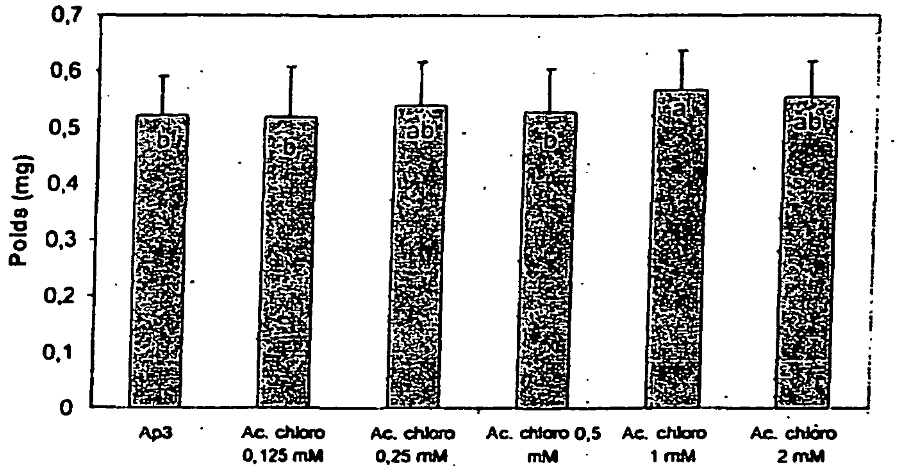
0,7
0,6
0,5
0,4
0,3
0,2
0,1
0
Poids (mg)
b
b
ab
b
a
ab
Ap3
Ac. chloro 0,125 mM
Ac. chloro 0,25 mM
Ac. chloro 0,5 mM
Ac. chloro 1 mM
Ac. chloro 2 mM

Figure 19

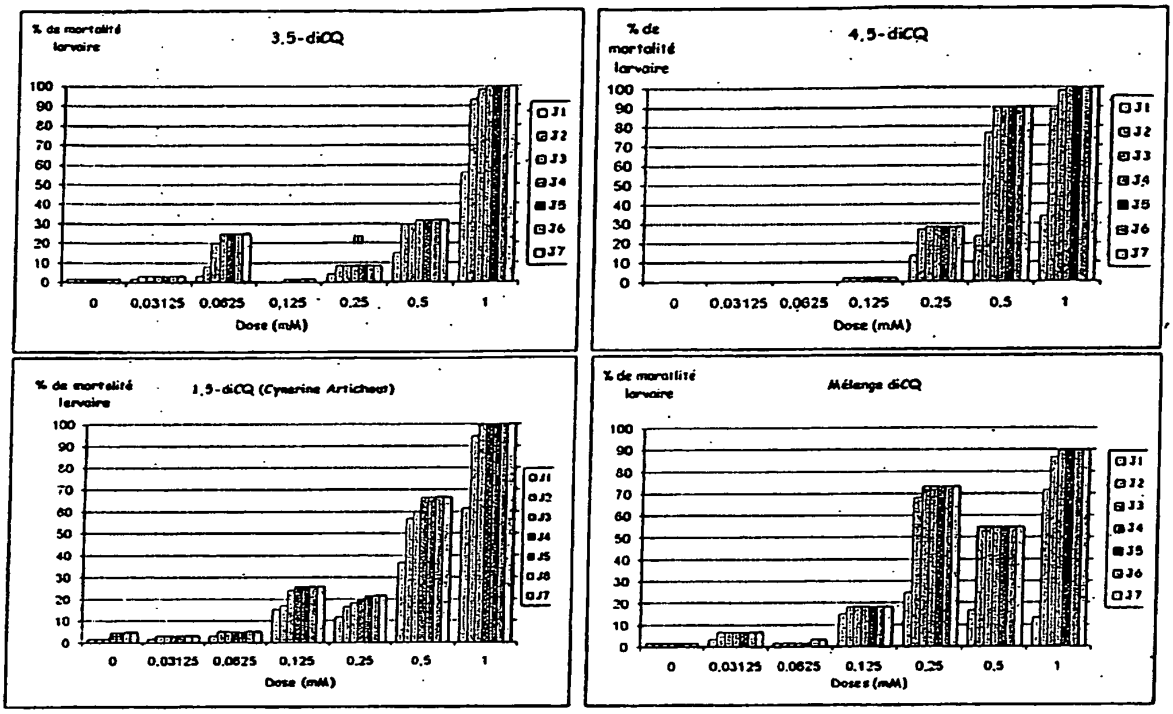
% de mortalité larvaire
3,5-diCQ
Dose (mM)
4,5-diCQ
1,5-diCQ (Cynarine Artichaut)
Mélange diCQ
Doses (mM)
J1
J2
J3
J4
J5
J6
J7

## Figure 20

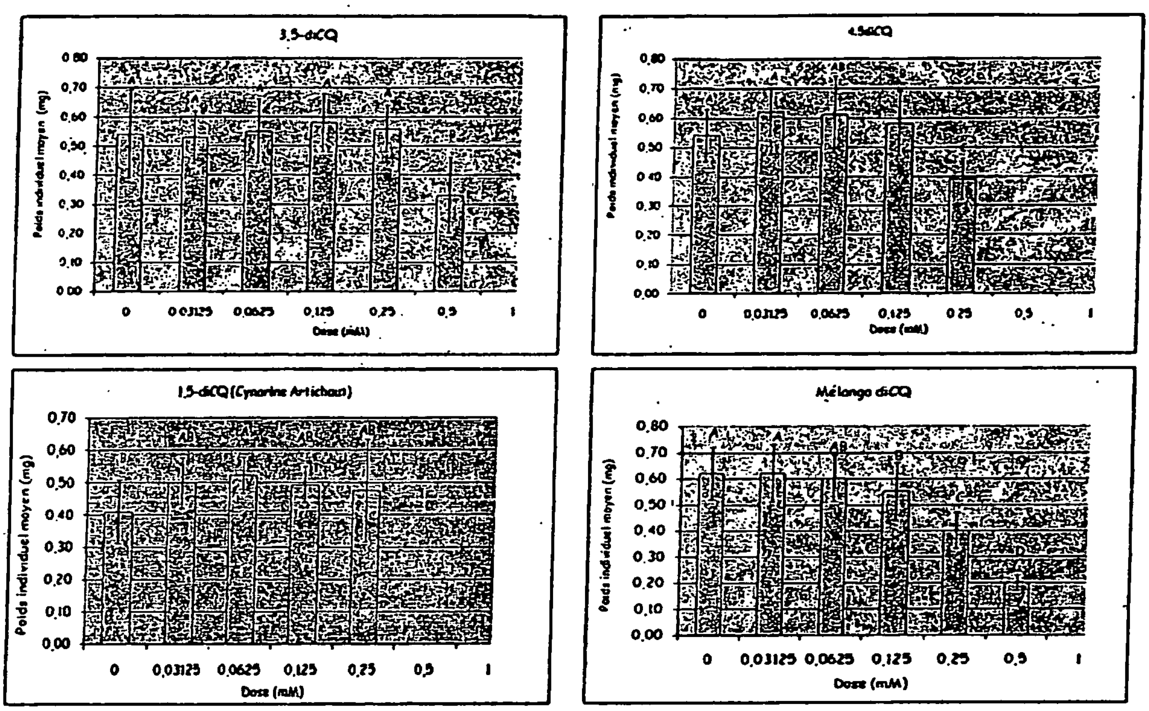
3,5-diCQ
1,5-diCQ (Cynarine Artichaut)
Mélange diCQ
Poids individuel moyen (mg)
0,00
0,10
0,20
0,30
0,40
0,50
0,60
0,70
0,80
0
0,03125
0,0625
0,125
0,25
0,5
1
Dose (mM)

# Figure 21

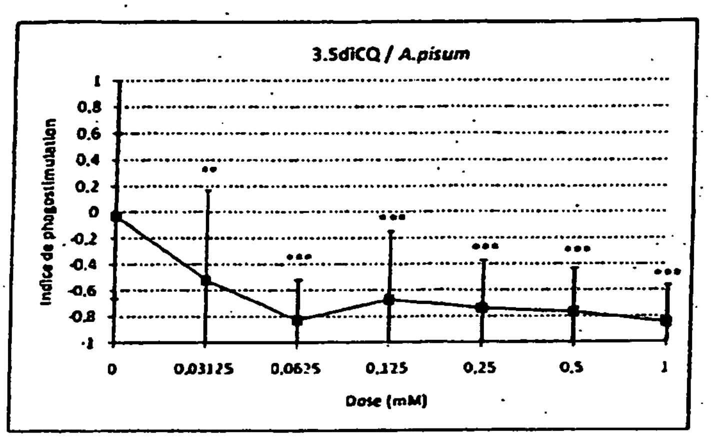
3.5diCQ / A.pisum
Indice de phagostimulation
1
0,8
0,6
0,4
0,2
0
-0,2
-0,4
-0,6
-0,8
-1
**
***
***
***
***
***
0
0,03125
0,0625
0,125
0,25
0,5
1
Dose (mM)

Figure 22

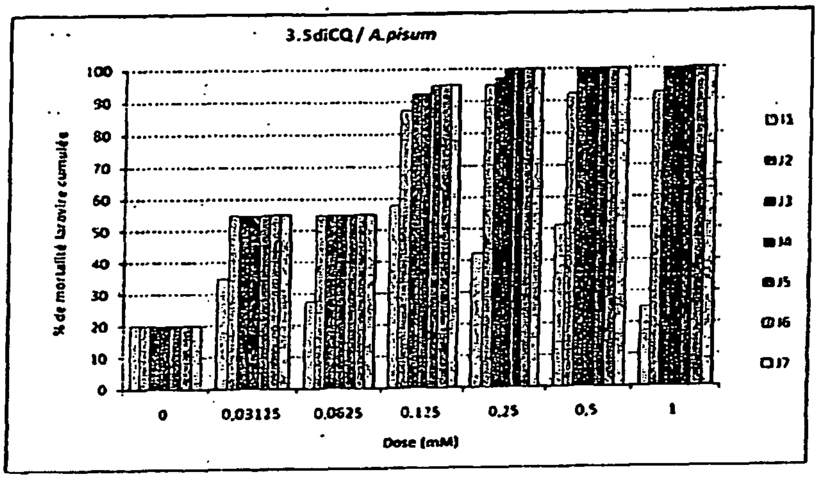
3.5diCQ / A.pisum
% de mortalité larvaire cumulée
100
90
80
70
60
50
40
30
20
10
0
0
0,03125
0,0625
0.125
0,25
0,5
1
Dose (mM)
J1
J2
J3
J4
J5
J6
J7

Figure 23

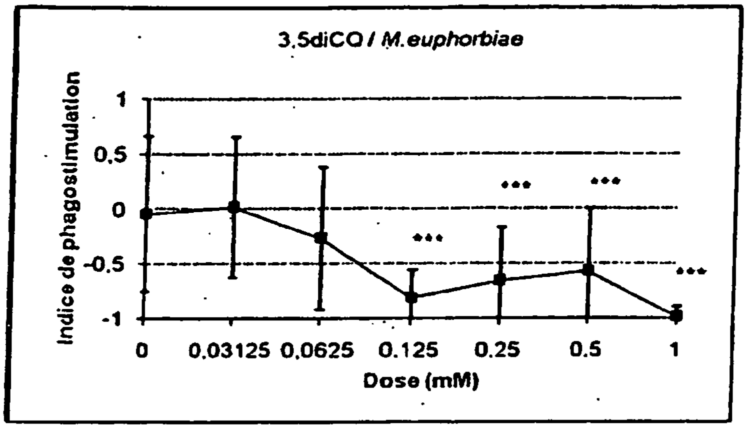
3,5diCQ / *M.euphorbiae*
Indice de phagostimulation
1
0,5
0
-0,5
-1
***
***
***
***
0
0,03125
0,0625
0.125
0.25
0.5
1
Dose (mM)

**Figure 24**

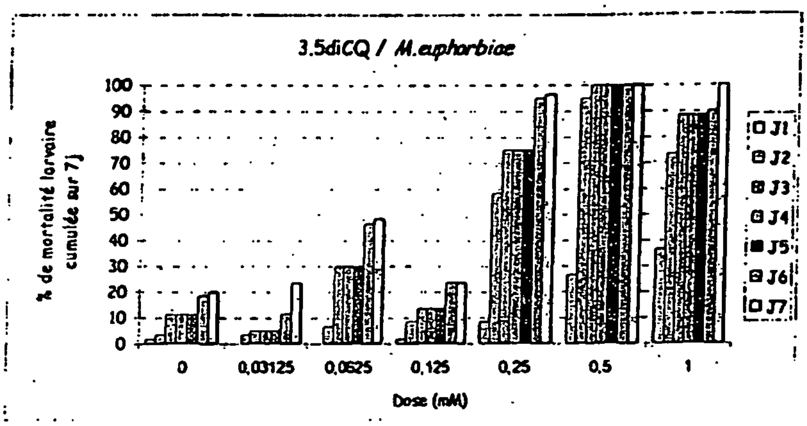
3.5diCQ / M.euphorbiae
% de mortalité larvaire cumulée sur 7J
100
90
80
70
60
50
40
30
20
10
0
0
0,03125
0,0625
0,125
0,25
0,5
1
Dose (mM)
J1
J2
J3
J4
J5
J6
J7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1008344 A
- WO 2006127525 A
- JP 2006213636 B
- EP 0577516 A
- EP 1312373 A
- EP 0299107 A
- EP 1671535 A
- WO 0133942 A


### Littérature non-brevet citée dans la description


- **OHNISHI et al.** *Phytochemistry,* 1994, vol. 36, 579-583
- **IWAI et al.** *Journal of Agricultural and Food Chemistry,* 2004, vol. 52, 4893-4898
- **KIM ; LEE.** *Planta Medica,* 2005, vol. 71, 871-876
- **SAITO et al.** *Bioorganic & Medicinal Chemistry,* 2005, vol. 13, 4191-4199
- **DOS SANTOS et al.** *Journal of Ethnopharmacology,* 2005, vol. 96, 545-549
- **MISHIMA et al.** *Biological & Pharmaceutical Bulletin,* 2005, vol. 28, 1909-1914
- **NGUYEN et al.** *Biological & Pharmaceutical Bulletin,* 2005, vol. 28, 2231-2234
- **BASNET et al.** *Biol Pharm Bull,* 1996, vol. 19, 1479-1484
- **PELUSO et al.** *Journal of Natural Products,* 1995, vol. 58, 639-646
- **MISHIMA et al.** *Bioorganic & Medicinal Chemistry,* 2005, vol. 13, 5814-5818
- **MAHMOOD et al.** *Antiviral Chem. Chemother.,* 1993, vol. 4, 235-240
- **MC DOUGALL et al.** *Antimicrobial Agents & Chemotherapy,* 1998, vol. 42, 140-146
- **ZHU et al.** *J Virol,* 1999, vol. 73, 3309-3316
- **BAZZALO et al.** *Phytopathologische Zeitschrift,* 1985, vol. 112, 322-332
- **KODOMA et al.** *Phytochemistry,* 1998, vol. 47, 371-373
- **STANGE et al.** *Postharvest Biology & Technology,* 2001, vol. 23, 85-92
- **COLE et al.** *Annals of Applied Biology,* 1984, vol. 105, 129-145
- **BENINGER et al.** *J Chem Ecol,* 2004, vol. 30, 589-605
- **MASSONIÉ et al.** Revue de zoologie agricole et pathologie végétale. 1979, vol. 78, 1-5
- **MONET ; MASSONIÉ.** *Agronomie,* 1994, vol. 2, 177-182
- **MONET ET GUYE.** Proc. Fourth Intern. Peach Symposium Acta Hort. 1998, 171-175
- *Nippon Shokuhin Kagaku Gakkaishi,* 2006, vol. 13 (3), 136-140
- *Biotechnology in Agriculture and Forestry,* 1993, vol. 21, 217-232
- *Journal of Herbs, Spices & Medicinal Plants,* 2002, vol. 10 (2), 73-81
- *Journal of Agricultural and Food Chemistry,* 1995, vol. 43 (10), 2592-2595
- *Phytochemistry,* 2008, vol. 69 (17), 2979-2983
- *Journal of Ethnopharmacology,* 2005, vol. 96 (3), 545-549
- **ISLAM et al.** *Journal of Agricultural and Food Chemistry,* 2002, vol. 50, 371-83722
- **ISLAM et al.** *Journal of Food Science,* 2003, vol. 68, 111-116
- **DE HANSON.** *Biochemistry,* 1965, vol. 4, 2719-2731
- **MOLLER ; HERRMANN.** *Journal of Chromatography,* 1982, vol. 241, 371-379
- **CHEN.** *Thèse de l'Institut National des Sciences Appliquées, Lyon, France,* 1996, 158
- **RAHBÉ ; FEBVAY.** *Entomologia Experimentalis et Applicata,* 1993, vol. 67, 149-160
- **CHEN.** *Thèse de l'Institut National des Sciences Appliquées, Lyon, France,* 1996, vol. 158
- **FEBVAY et al.** *Canadian Journal of Zoology,* 1988, vol. 66, 2149-2453